# EUROPEAN PATENT APPLICATION

(11) **EP 2 107 122 A1**
(43) Date of publication of application: **07.10.2009**
(21) Application number: 09004536.0
(22) Date of filing: 30.03.2009
(51) Int. Cl.: C12Q 1/37

(54) **Protease detection material, set of protease detection materials, and method for measuring protease**

(30) Priority: 31.03.2008 JP 2008094272
(71) Applicant: FUJIFILM Corporation, Minato-ku Tokyo (JP)
(72) Inventor: Shuto, Sadanobu, Kanagawa (JP); Ohzeki, Katsuhisa, Minami-Ashigara-shi Kanagawa (JP); Mori, Mikinaga, Ashigarakami-gun Kanagawa (JP)
(74) Representative: Siegert, Georg

(57) **Abstract**

The present invention provides a protease detection material having, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance. Further, a set of protease detection materials and a method for measuring protease using the above-described protease detection material are provided. A protease detection material, a set of protease detection materials, and a method of measuring protease which allow protease activity to be measured promptly with high sensitivity, and that allow, at the same time, morphological observation of tissues and cells on a thin film to be performed easily.

## Description

### BACKGROUND OF THE INVENTION

### Field of the Invention

The present invention relates to a protease detection material, a set of protease detection materials, and a method for measuring protease. More specifically, it relates to a protease detection material, a set of protease detection materials, and a method for measuring protease that make it possible to accurately diagnose the degree of malignancy of cancers such as invasion activity and metastasis activity of cancer cells, the degree of progress in periodontal diseases such as periodontitis, and destructive lesion in rheumatic arthritis or the like.

### Description of the Related Art

Various proteases such as matrix metalloproteases and plasminogen activators are known to participate in infiltration and metastasis of cancer cells, progress in periodontal diseases such as periodontitis, progress in tissue destruction such as that in rheumatoid arthritis, healing processes in wounds, ontogeny processes, and the like. As methods for detecting and quantitatively measuring such proteases, immunoassay methods and immunoblotting methods using antibodies, electrophoretic zymography methods, and the like are known. Further, as a method for measuring protease activity in tissues, a so-called in situ zymography method described in FASEB Journal, vol. 9, July, pages 974 to 980 (1995) and International Patent Publication (WO) No. 97/32035 is known. In Japanese Patent Application Laid-Open (JP-A) No. 2000-325096, staining a trace of digestion of a thin film of gelatin or the like with a color such as red, orange or yellow to identify a cell having protease activity is disclosed.

### SUMMARY OF THE INVENTION

The present invention has been made in view of the above circumstances and provides a protease detection material, a set of protease detection materials and a method for measuring protease with the following aspects.

A first aspect of the invention provides a protease detection material comprising, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance.

A second aspect of the invention provides a method for measuring protease using a protease detection material comprising, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance, the method comprising:
(1) bringing a biological test sample into contact with a surface of the protease detection material; and
(2) causing a developing agent to act on the surface of the protease detection material having been subjected to (1) to detect a trace of digestion by protease.

A third aspect of the invention provides a set of protease detection materials comprising:
a first protease detection material having, on a first support, a first layer containing a dye precursor and a layer which contains a protease substrate and is disposed on the same side of the first support as the first layer containing the dye precursor and farther from the first support than the first layer containing the dye precursor, wherein the layer containing the protease substrate further contains a first development center-forming substance; and
a second protease detection material for reference having, on a second support, at least a second layer containing a dye precursor and a layer which contains a protease inhibitor for an object to be detected and is disposed on the same side of the second support as the second layer containing the dye precursor and farther from the second support than the second layer containing the dye precursor, wherein the layer containing the protease inhibitor for an object to be detected further contains the second development center-forming substance.

A fourth aspect of the invention provides a method for measuring protease using the set of protease detection materials according to the third aspect, the method comprising:
(1) bringing one slice among two or more substantially continuous slices of a biological test sample into contact with a surface of the first protease detection material;
(2) bringing another slice among the two or more continuous slices into contact with a surface of the second protease detection material for reference;
(3) causing a color developing agent to act on the surface of the first protease detection material having been subjected to (1) and on the surface of the second protease detection material for reference having been subjected to (2), to detect a trace of digestion by protease by means of color-formation pattern; and
(4) comparing the color-formation pattern of the first protease detection material with the color-formation pattern of the second protease detection material for reference.

### DETAILED DESCRIPTION OF THE INVENTION

The present inventors conducted earnest research on the in situ zymography method and the application thereof, and, in the process of the research, recognized such problems that, although a method, in which a trace of digestion of a thin film of gelatin or the like is stained with a color such as red, orange or yellow for measuring protease activity to identify cells having protease activity, makes the observation of tissues and cells on the thin film possible relatively easily, the sensitivity is not sufficient and does not allow cells having protease activity for initial symptoms at micro stages to be detected.

Accordingly, an object of the invention is to provide a means for solving these problems. More specifically, it is to provide a protease detection material, a set of protease detection materials and a method for measuring protease that allow protease activity to be measured promptly with high sensitivity, and that allow, at the same time, morphological observation of tissues and cells on a thin film to be performed easily.

The problem of the invention described above has been solved by a protease detection material having, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance.

Preferably, the development center-forming substance is silver colloid, gold colloid, or silver halide.
Preferably, a thickness of the layer containing the protease substrate is 3 µm or less.
Preferably, the layer containing the protease substrate further contains a hardener.
Preferably, the protease detection material further includes a color developing agent.
Preferably, the color developing agent is a compound represented by the following formula (I).

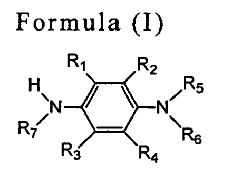

In formula (I), R₁, R₂, R₃ and R₄ each independently represent a hydrogen atom or a substituent; R₅ and R₆ each independently represent an alkyl group, an aryl group, a heterocyclic group, an acyl group, or a sulfonyl group; R₁ and R₂, R₃ and R₄, R₅ and R₆, R₂ and R₅, or R₄ and R₆ may bond to each other in each combination to form a 5-, 6-, or 7-membered ring; R₇ represents a hydrogen atom, R₁₁-O-CO-, R₁₂-CO-CO-, R₁₃-NH-CO-, R₁₄-SO₂-, R₁₅-W-C(R₁₆)(R₁₇)(R₁₈)-, R₁₉-SO₂NHCO-, R₂₀-CONHCO-, R₂₁-SO₂NHSO₂-, R₂₂-CONHSO₂-, or (M)_{1/n}OSO₂-; R₁₁, R₁₂, R₁₃, R₁₄, R₁₉, R₂₀, R₂₁, and R₂₂ each independently represent an alkyl group, an aryl group, or a heterocyclic group; R₁₅ represents a hydrogen atom or a block group; W represents an oxygen atom, a sulfur atom, or -N(R₁₈)-; R₁₆, R₁₇, and R₁₈ each independently represent a hydrogen atom or an alkyl group; and M represents a cation having a valency of n.

Preferably, the color developing agent is a compound represented by the following formula (II).

Formula (II) R¹¹-NH-NH-X-R¹²

In formula (II), R¹¹ represents an aryl group or a heterocyclic group, each of which may have a substituent; R¹² represents an alkyl group, an alkenyl group, an alkynyl group, an aryl group, or a heterocyclic group, each of which may have a substituent; X represents -SO₂-, -CO-, -COCO-, -CO-O-, -CO-N(R¹³)-, -COCO-O-, - COCO-N(R¹³)-, or -SO₂-N(R¹³)-; and R¹³ represents a hydrogen atom or a group selected from the groups that may be represented by R¹².

Preferably, the color developing agent is a compound represented by the following formula (IV).

Formula (IV) R¹¹-NH-NH-CO-R¹²

In formula (IV), R¹¹ and R¹² each have the same meaning as that in formula (II).

Preferably, the dye precursor is at least one compound represented by a formula selected from the group consisting of the following formulae (C-1), (C-2), (C-3), (M-1), (M-2), (M-3), (Y-1), (Y-2), and (Y-3).

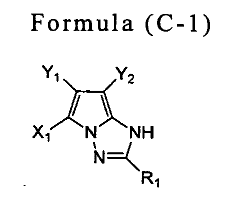

In formula (C-1), X₁ represents a hydrogen atom or a leaving group, Y₁ and Y₂ each independently represent an electron-attracting substituent, and R₁ represents an alkyl group, an aryl group, or a heterocyclic group.

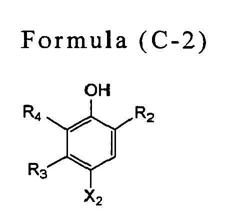

In formula (C-2), X₂ represents a hydrogen atom or a leaving group, R₂ represents an acylamino group, a ureido group, or a urethane group, R₃ represents a hydrogen atom, an alkyl group, or an acylamino group, R₄ represents a hydrogen atom or a substituent, and R₃ and R₄ may link together to form a ring.

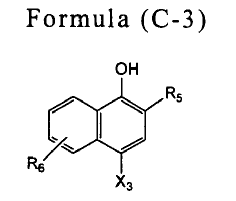

In formula (C-3), X₃ represents a hydrogen atom or a leaving group, R₅ represents a carbamoyl group or a sulfamoyl group, and R₆ represents a hydrogen atom or a substituent.

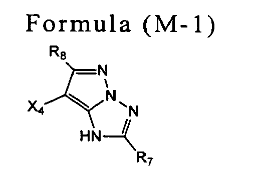

In formula (M-1), X₄ represents a hydrogen atom or a leaving group, R₇ represents an alkyl group, an aryl group, or a heterocyclic group, and R₈ represents a substituent.

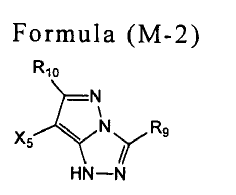

In formula (M-2), X₅ represents a hydrogen atom or a leaving group, R₉ represents an alkyl group, an aryl group, or a heterocyclic group, and R₁₀ represents a substituent.

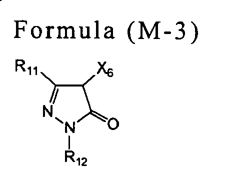

In formula (M-3), X₆ represents a hydrogen atom or a leaving group, R₁₁ represents an alkyl group, an aryl group, an acylamino group, or an anilino group, and R₁₂ represents an alkyl group, an aryl group, or a heterocyclic group.

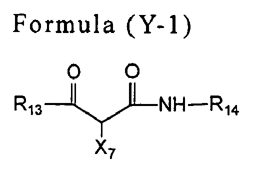

In formula (Y-1), X₇ represents a hydrogen atom or a leaving group, R₁₃ represents an alkyl group, an aryl group, or an indolenyl group, and R₁₄ represents an aryl group or a heterocyclic group.

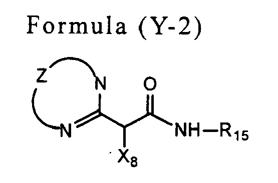

In formula (Y-2), X₈ represents a hydrogen atom or a leaving group, Z represents a divalent group necessary for forming a 5- to 7-membered ring, and R₁₅ represents an aryl group or a heterocyclic group.

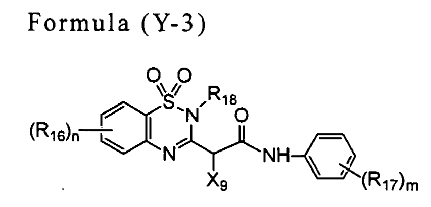

In formula (Y-3), X₉ represents a hydrogen atom or a leaving group, R₁₆, R₁₇, and R₁₈ each independently represent a substituent, n represents an integer of from 0 to 4, and m represents an integer of from 0 to 5. When n represents 2 or more, a plurality of R₁₆ may be the same or different from one another, and when m represents 2 or more, a plurality of R₁₇ may be the same or different from one another.

The method for measuring protease of the invention is a method for measuring protease using a protease detection material having, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance, the method including:
(1) bringing a biological test sample into contact with a surface of the protease detection material; and
(2) causing a developing agent to act on the surface of the protease detection material having been subjected to (1) to detect a trace of digestion by protease.

The set of protease detection materials comprising:
a first protease detection material having, on a first support, a first layer containing a dye precursor and a layer which contains a protease substrate and is disposed on the same side of the first support as the first layer containing the dye precursor and farther from the first support than the first layer containing the dye precursor, wherein the layer containing the protease substrate further contains a first development center-forming substance; and
a second protease detection material for reference having, on a second support, at least a second layer containing a dye precursor and a layer which contains a protease inhibitor for an object to be detected and is disposed on the same side of the second support as the second layer containing the dye precursor and farther from the second support than the second layer containing the dye precursor, wherein the layer containing the protease inhibitor for an object to be detected further contains a second development center-forming substance.

The method for measuring protease using the set of protease detection materials described above, the method comprising:
(1) bringing one slice among two or more substantially continuous slices of a biological test sample into contact with a surface of the first protease detection material;
(2) bringing another slice among the two or more continuous slices into contact with a surface of the second protease detection material for reference;
(3) causing a color developing agent to act on the surface of the first protease detection material having been subjected to (1) and on the surface of the second protease detection material for reference having been subjected to (2), to detect a trace of digestion by protease by means of color-formation pattern; and
(4) comparing the color-formation pattern of the first protease detection material with the color-formation pattern of the second protease detection material for reference.

In the present invention, a first support and a second support, a dye precursor contained in a first layer containing a dye precursor and a dye precursor contained in a second layer containing a dye precursor, and a first development center-forming substance and a second development center-forming substance may be each independently the same or different from each other.

According to the method of the invention, the trace of digestion can be observed promptly with high sensitivity, and, at the same time, tissues or cells on a thin film can be observed promptly with high sensitivity, to make the identification of tissue parts or cells that exhibit protease activity easy. For example, by staining a thin film and cell nuclei at the same time, morphological information of the cell nuclei and the trace of digestion can be observed using the same sample. Therefore, it is possible to determine simply and easily whether an abnormal cell that has generated morphological change exhibits protease activity.

The protease detection material of the present invention has, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance.

Preferably, the development center-forming substance is silver colloid, gold colloid, or silver halide.

After bringing a slice of a biological test sample into contact with a surface of the protease detection material to form a trace of protease digestion in the layer containing the protease substrate, a peroxide and a color developing agent are caused to act on the surface. Then, the development center-forming substance serves as a nucleus, and an oxidation-reduction reaction proceeds, to form a dye by the reaction between an oxidation product of the color developing agent and the dye precursor. Since the color development by the peroxide using the development center-forming substance as a nucleus proceeds through a chain reaction, the trace of digestion is amplified to give a color-formation pattern with a distinct contrast. Consequently, a slight trace of digestion, which has been conventionally difficult to detect, can also be detected promptly with high sensitivity.

In the invention, the layer containing the protease substrate is a thin layer having a thickness of 3 µm or less, and preferably 1 µm or less. More preferably, the layer containing the protease substrate further contains a hardener.

It is preferred that the protease detection material of the present invention further has a layer containing a color developing agent.

Preferably, the color developing agent is a compound represented by the above formula (I), or a compound represented by the above formula (II). More preferably, the color developing agent is a compound represented by the above formula (II) or (IV).

Preferably, the dye precursor is at least one compound represented by a formula selected from the group consisting of the above formulae (C-1), (C-2), (C-3), (M-1), (M-2), (M-3), (Y-1), (Y-2), and (Y-3).

Another embodiment of the present invention is a set of protease detection materials including a first protease detection material and a second protease detection material for reference.

The first protease detection material has, on a first support, at least a first layer containing a dye precursor and a layer which contains a protease substrate and is disposed on the same side of the first support as the first layer containing the dye precursor and farther from the first support than the first layer containing the dye precursor, wherein the layer containing the protease substrate further contains a first development center-forming substance. Further, the second protease detection material for reference has, on a second support, at least a second layer containing a dye precursor and a layer which contains a protease inhibitor for an object to be detected and is disposed on the same side of the second support as the second layer containing the dye precursor and farther from the second support than the second layer containing the dye precursor, wherein the layer containing the protease inhibitor for an object to be detected further contains a second development center-forming substance.

The method for measuring protease using the set of protease detection materials described above includes: (1) bringing one slice among two or more substantially continuous slices of a biological test sample into contact with a surface of the first protease detection material; (2) bringing another slice among the two or more continuous slices into contact with a surface of the second protease detection material for reference; (3) causing a color developing agent to act on the surface of the first protease detection material having been subjected to (1) and on the surface of the second protease detection material for reference having been subjected to (2), to detect a trace of digestion by protease by means of color-formation pattern; and (4) comparing a color-formation pattern of the first protease detection material with a color-formation pattern of the second protease detection material for reference.

The protease inhibitor is preferably a chelating agent, a matrix metalloprotease inhibitor, or a serine protease inhibitor.
By using the protease detection material for reference in parallel with the protease detection material, a protease that is to be an object of detection can be detected with higher precision.

According to the above-described preferable embodiment of the invention, the above-described method, wherein the biological sample is a biological sample separated/collected from a mammal which may include a human, and preferably from a patient, a mammal which is suspected of illness, a test animal or the like, is provided. As the biological sample, in addition to solid samples such as tissue slices, samples containing cells or tissue slices collected from a tissue by suction, and nonsolid samples such as blood, lymph and saliva can be used. In a method in which a continuous slice is used, a tissue slice can be used as a biological sample.

For detecting the trace of digestion, such a method can be employed as visually determining the color-formation pattern using a microscope, or quantifying or digitizing it using an image-processing apparatus.

The term "measuring method" used herein should be construed in its widest sense, including qualitative and quantitative methods. The method for measuring protease of the invention is **characterized in that**, in a method in which protease is measured by allowing the protease contained in a sample to digest a protease substrate to form a trace of digestion in a thin film (the so-called in situ zymography method disclosed, for example, in WO No. 97/32035), the thin film in which the trace of digestion has been formed is converted to a color-formation pattern that has been amplified utilizing a specified color-formation reaction, and the color-formation pattern is observed. The method of the invention is **characterized in that** morphological observation of a biological sample on the thin film can easily be performed, as well as in that it is possible to detect the trace of digestion on the thin film as a portion having a low dye density and definitely prove the existence of protease activity in the sample. As the thin film for use in the method of the invention, those described in WO No. 97/32035 can be employed. As the hardener or the protease inhibitor, those described in the aforementioned document can also be employed.

Examples of the protease to be the object of the present invention include matrix metalloproteases and serine proteases.
These enzymes are described in detail in "Molecular Mechanism of Cancer Metastasis (Gan Tenn'i no Bunshi Kiko)" edited by Tsuruo Takashi, pages 92 to 107, MEDICAL VIEW CO., LTD, published in 1993. Examples of proteases particularly suitable for the methods of the invention include matrix metalloproteases such as interstitial collagenase (MMP-1), gelatinase A (MMP-2) and gelatinase B (MMP-9); and serine proteases such as plasminogen activator (PA).
However, the objects of the methods of the invention are not limited to the above-described specific proteases.

The protease substrate is not particularly limited so long as it is a high-molecular compound that is degradable as a protease substrate. For example, collagen, gelatin, proteoglycan, fibronectin, laminin, elastin or casein may be used. Preferably, collagen, gelatin, fibronectin or casein is used, and gelatin or casein is more preferably used. When gelatin is used, a type of gelatin is not particularly limited, and for example, alkali-processed bovine bone gelatin, alkali-processed swine cutis gelatin, acid-processed bovine bone gelatin, phthalated bovine bone gelatin, acid-processed swine cutis gelatin, or the like can be used. The protease substrate may be used alone, or two or more of the above-described substances may be used in combination.

There may be cases where the use of two or more different protease substrates in combination allows the type of protease contained in the biological sample to be identified accurately. For example, it is possible to bring one of substantially continuous slices of a biological sample into contact with a thin film containing a certain type of protease substrate to detect a trace of digestion, and to bring another slice into contact with a thin film containing a different protease substrate to detect a trace of digestion on the thin film, and then to compare the respective results. Two or more thin films containing different protease substrates, respectively, may be used for comparison. A thickness of the layer containing a protease substrate is 3 µm or less, and preferably 1 µm or less.

As biological samples used for the methods of the invention, those separated/collected from a mammal including a human can be employed. For example, biological samples separated/collected from diseased mammals, mammals in which the existence of a disease is suspected, or test animals can be used. The form of the biological sample is not particularly limited, and solid samples such as a tissue slice and nonsolid samples such as body fluid can be used. Examples of usable nonsolid samples include samples containing cells or tissue slices collected by suction from a tissue, and body fluids such as blood, lymph and saliva. For example, cancer tissues separated/collected by surgical operation or histological examination from tumor tissues such as lung cancer, stomach cancer, esophageal cancer, colon cancer, breast cancer, uterine cancer, ovarian cancer, thyroid cancer, liver cancer, intraoral cancer, prostatic cancer, renal cancer or bladder cancer; tissues separated/collected from tissues such as lymph nodes, tissues of periodontal diseases, synovial membranes and bone tissues of rheumatic arthritis and atherosclerotic tissues by surgical operation or histological examination; gingival crevicular fluids, fluids contained in destructive morbid tissues (for example, synovial fluid of rheumatic morbidity, extracted fluid of alveolar pyorrhea tissue), pleural effusions, ascites, cerebrospinal fluids, mammary gland abnormal secretion fluids, ovarian retention fluids, sputum or the like can be used.

When the sample is a tissue, for example, a slice having a thickness of from 1 µm to 10 µm, and preferably from 4 µm to 6 µm, may be prepared from a sample rapidly frozen in liquid nitrogen by using an apparatus for preparing a frozen slice, and then the slice may be applied to a thin film so as to bring the sample into contact with the thin film. A nonsolid sample containing cells or tissue slices collected by paracentesis and suction may be mixed with a forming material such as a compound, rapidly forzen with liquid nitrogen, and made into slices in a similar manner for use. When a nonsolid sample containing cells or tissue slices collected from a tissue by paracentesis and suction is used directly, the sucked sample may be discharged on a thin film to allow cells to adhere to the thin film in a dispersion state. Further, when the biological sample is a tissue slice, moisture of the collected tissue may be gently wiped, and then the tissue can be left at rest on a thin film containing a protease substrate for about 1 minute to about 30 minutes to allow the sample to be in contact with the thin film.

When a nonsolid sample such as synovial fluid collected from a patient with rheumatoid arthritis is used, the sample may be diluted to an appropriate concentration, and/or subjected to a necessary pretreatment, and then the sample in an amount of from about 1 µL to about 50 µL, or preferably from about 1 µL to about 20 µL, may be dropped onto the thin film. When gingival crevicular fluid of periodontal disease is used as a sample, it is possible to adopt a method in which a piece of filter paper is inserted into a gingival crevice to collect about 5 µL to about 10 µL of gingival crevicular fluid, and then the filter paper is attached to the thin film. After the collection of gingival crevicular fluid, the gingival crevicular fluid may be extracted from the filter paper using distilled water or a suitable buffer solution (for example, 50 mM Tris-HCl, pH 7.5, 10 mM CaCl₂, 0.2 M NaCl, or the like) according to need, and the extract may be dropped onto the thin film.

After a sample containing protease is brought into contact with the thin film by such a means as attaching a tissue slice containing a protease substrate to the thin film or dropping a liquid sample, the membrane is incubated at a temperature suitable for exhibition of protease activity, for example, at 37°C, under a saturated humid condition for a period required for digestion of the substrate. Time required for incubation may vary depending on the type of the sample or thin film, and the incubation is performed for 10 minutes to 24 hours, and preferably for 20 minutes to 6 hours at 37°C, to form traces of digestion, by protease in the sample, in the thin film.

Thereafter, a peroxide and a color developing agent are caused to act to bring about a color-formation reaction using a development center-forming substance as a nucleus, thereby forming a dye through the reaction between an oxidation product of a color developing agent and a dye precursor. The dye is formed in a thin film that remains without being digested, and traces of digestion are not subjected to color formation. Consequently, the traces of digestion are observed as patterns having no color against a color-formed background.

Further, in order to observe specific protease serving as an object to be detected with high precision, one of two or more substantially continuous slices in a biological sample is attached to a thin film containing no protease inhibitor, and another slice is attached to a thin film containing a protease inhibitor. Then, by comparing traces of digestion in the two films, identification of the type of protease is possible.

Next, the development center-forming substance, color developing agent and dye precursor, which are used for color formation in the invention, are to be explained.

### 1) Development center-forming substance

The development center-forming substance used in the present invention is a substance which becomes a catalyst that works for an oxidation-reduction reaction of hydrogen peroxide and a color developing agent, and is sometimes called as a catalytic nucleus. Preferably a metal colloid or a silver halide grain is used.

As the silver halide grain, silver chloride, silver bromide, silver iodide, silver chlorobromide, silver bromoiodide or silver chlorobromoiodide, which are used in silver halide photographic materials, can be used.

As the metal colloid, any metal colloid can be used as long as it works as a catalytic nucleus for the oxidation-reduction reaction described above. Specific examples of the metal colloid include colloids of gold, silver, platinum, palladium, lead, zinc, cadmium, tin, chromium, copper or cobalt, and mixtures thereof; and preferably include colloids of gold, silver, platinum or palladium, and mixtures thereof. Among them, particularly preferable are gold colloid and silver colloid.

A mean particle diameter of the metal colloid is preferably from 1 nm to 500 nm, and more preferably from 5 nm to 100 nm.

### 2) Color developing agent

A preferable embodiment of the color developing agent used in the present invention is a compound represented by the following formula (I).

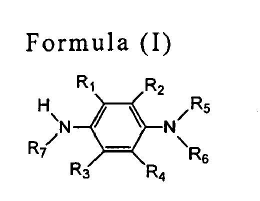

In formula (I), R₁, R₂, R₃ and R₄ each independently represent a hydrogen atom or a substituent; R₅ and R₆ each independently represent an alkyl group, an aryl group, a heterocyclic group, an acyl group, or a sulfonyl group; R₁ and R₂, R₃ and R₄, R₅ and R₆, R₂ and R₅, or R₄ and R₆ may bond to each other in each combination to form a 5-, 6-, or 7-membered ring; R₇ represents a hydrogen atom, R₁₁-O-CO-, R₁₂-CO-CO-, R₁₃-NH-CO-, R₁₄-SO₂-, R₁₅-W-C(R₁₆)(R₁₇)(R₁₈)-, R₁₉-SO₂NHCO-, R₂₀-CONHCO-, R₂₁-SO₂NHSO₂-, R₂₂-CONHSO₂-, or (M)_{1/n}OSO₂-; R₁₁, R₁₂, R₁₃, R₁₄, R₁₉, R₂₀, R₂₁, and R₂₂ each independently represent an alkyl group, an aryl group, or a heterocyclic group; R₁₅ represents a hydrogen atom or a block group; W represents an oxygen atom, a sulfur atom, or -N(R₁₈)-; R₁₆, R₁₇, and R₁₈ each independently represent a hydrogen atom or an alkyl group; and M represents a cation having a valency of n.

The compound represented by formula (I) according to the invention will be explained in detail. R₁, R₂, R₃, and R₄ each independently represent a hydrogen atom or a substituent. Examples of the substituent represented by R₁, R₂, R₃, and R₄ include a halogen atom, an alkyl group (including a cycloalkyl group and a bicycloalkyl group), an alkenyl group (including a cycloalkenyl group and a bicycloalkenyl group), an alkynyl group, an aryl group, a heterocyclic group, a cyano group, a hydroxy group, a nitro group, a carboxy group, an alkoxy group, an aryloxy group, silyloxy group, a heterocyclic oxy group, an acyloxy group, a carbamoyloxy group, an alkoxycarbonyloxy group, an aryloxycarbonyloxy group, an amino group (including an anilino group), an acylamino group, an aminocarbonylamino group, an alkoxycarbonylamino group, an aryloxycarbonylamino group, a sulfamoylamino group, an alkylsulfonylamino group, an arylsulfonylamino group, a mercapto group, an alkylthio group, an arylthio group, a heterocyclic thio group, a sulfamoyl group, a sulfo group, an alkylsulfinyl group, an arylsulfinyl group, an alkylsulfonyl group, an arylsulfonyl group, an acyl group, an aryloxycarbonyl group, an alkoxycarbonyl group, a carbamoyl group, an arylazo group, a heterocyclic azo group, an imido group, a phosphino group, a phosphinyl group, a phosphinyloxy group, a phosphinylamino group, and a silyl group. Further in detail, a halogen atom (for example, a chlorine atom, a bromine atom, or an iodine atom), an alkyl group [which represents a substituted or unsubstituted, linear, branched, or cyclic alkyl group; an alkyl group (preferably, an alkyl group having 1 to 30 carbon atoms; for example, methyl, ethyl, n-propyl, isopropyl, t-butyl, n-octyl, eicosyl, 2-chloroethyl, 2-cyanoethyl, and 2-ethylhexyl), a cycloalkyl group (preferably, a substituted or unsubstituted cycloalkyl group having 3 to 30 carbon atoms; for example, cyclohexyl, cyclopentyl, and 4-n-dodecylcyclohexyl), a bicycloalkyl group (preferably, a substituted or unsubstituted bicycloalkyl group having 5 to 30 carbon atoms, namely, it means a monovalent group obtained by removing one hydrogen atom from bicycloalkane having 5 to 30 carbon atoms; for example, bicyclo[1,2,2]heptan-2-yl, bicyclo[2,2,2]octan-3-yl), and further a tricyclo structure having many cyclic structures, and the like are included; an alkyl group included in a substituent described below (for example, an alkyl group in an alkylthio group) also represents the alkyl group of this concept], an alkenyl group [which represents a substituted or unsubstituted, linear, branched, or cyclic alkenyl group; an alkenyl group (preferably, an alkenyl group having 2 to 30 carbon atoms; for example, vinyl, allyl, prenyl, gelanyl, and oleyl), a cycloalkenyl group (preferably, a substituted or unsubstituted cycloalkenyl group having 3 to 30 carbon atoms, namely, it means a monovalent group obtained by removing one hydrogen atom from cycloalkene having 3 to 30 carbon atoms; for example, 2-cyclopenten-1-yl and 2-cyclohexen-1-yl), a bicycloalkenyl group (a substituted or unsubstituted bicycloalkenyl group, and preferably, a substituted or unsubstituted bicycloalkenyl group having 5 to 30 carbon atoms, namely, it means a monovalent group obtained by removing one hydrogen atom from bicycloalkene having one double bond; for example, bicyclo[2,2,1]hepto-2-en-1-yl, bicyclo[2,2,2]octo-2-en-4-yl) are described], an alkynyl group (preferably, a substituted or unsubstituted alkynyl group having 2 to 30 carbon atoms; for example, ethynyl, propargyl, and a trimethylsilylethynyl group), an aryl group (preferably, a substituted or unsubstituted aryl group having 6 to 30 carbon atoms; for example, phenyl, p-tolyl, naphthyl, m-chlorophenyl, and o-hexadecanoylaminophenyl), a heterocyclic group (preferably, a monovalent group obtained by removing one hydrogen atom from a 5- or 6-membered, substituted or unsubstituted, aromatic or non-aromatic heterocyclic compound, more preferably, a 5- or 6-membered aromatic heterocyclic group having 3 to 30 carbon atoms; for example, 2-furyl, 2-thienyl, 2-pyrimidinyl, and 2-benzothiazolyl), a cyano group, a hydroxy group, a nitro group, a carboxy group, an alkoxy group (preferably, a substituted or unsubstituted alkoxy group having 1 to 30 carbon atoms; for example, methoxy, ethoxy, isopropoxy, t-butoxy, n-octyloxy, and 2-methoxyethoxy), an aryloxy group (preferably, a substituted or unsubstituted aryloxy group having 6 to 30 carbon atoms; for example, phenoxy, 2-methylphenoxy, 4-t-butylphenoxy, 3-nitrophenoxy, and 2-tetradecanoylaminophenoxy), a silyloxy group (preferably, a silyloxy group having 3 to 20 carbon atoms; for example, trimethylsilyloxy and t-butyldimethylsilyloxy), a heterocyclic oxy group (preferably, a substituted or unsubstituted heterocyclic oxy group having 2 to 30 carbon atoms; for example, 1-phenyltetrazole-5-oxy and 2-tetrahydropyranyloxy), an acyloxy group (preferably, a formyloxy group, a substituted or unsubstituted alkylcarbonyloxy group having 2 to 30 carbon atoms, or a substituted or unsubstituted arylcarbonyloxy group having 6 to 30 carbon atoms; for example, formyloxy, acetyloxy, pivaloyloxy, stearoyloxy, benzoyloxy, and p-methoxyphenylcarbonyloxy), a carbamoyloxy group (preferably, a substituted or unsubstituted carbamoyloxy group having 1 to 30 carbon atoms; for example, N,N-dimethylcarbamoyloxy, N,N-diethylcarbamoyloxy, morpholinocarbonyloxy, N,N-di-n-octylaminocarbonyloxy, and N-n-octylcarbamoyloxy), an alkoxycarbonyloxy group (preferably, a substituted or unsubstituted alkoxycarbonyloxy group having 2 to 30 carbon atoms; for example, methoxycarbonyloxy, ethoxycarbonyloxy, t-butoxycarbonyloxy, and n-octylcarbonyloxy), an aryloxycarbonyloxy group (preferably, a substituted or unsubstituted aryloxycarbonyloxy group having 7 to 30 carbon atoms; for example, phenoxycarbonyloxy, p-methoxyphenoxycarbonyloxy, and p-n-hexadecyloxyphenoxycarbonyloxy), an amino group (preferably, an amino group, a substituted or unsubstituted alkylamino group having 1 to 30 carbon atoms, or a substituted or unsubstituted anilino group having 6 to 30 carbon atoms; for example, amino, methylamino, dimethylamino, anilino, N-methyl-anilino, and diphenylamino), an acylamino group (preferably, a formylamino group, a substituted or unsubstituted alkylcarbonylamino group having 1 to 30 carbon atoms, or a substituted or unsubstituted arylcarbonylamino group having 6 to 30 carbon atoms; for example, formylamino, acetylamino, pivaloylamino, lauroylamino, benzoylamino, and 3,4,5-tri-n-octyloxyphenylcarbonylamino), an aminocarbonylamino group (preferably, a substituted or unsubstituted aminocarbonylamino group having 1 to 30 carbon atoms; for example, carbamoylamino, N,N-dimethylaminocarbonylamino, N,N-diethylaminocarbonylamino, and morpholinocarbonylamino), an alkoxycarbonylamino group (preferably, a substituted or unsubstituted alkoxycarbonylamino group having 2 to 30 carbon atoms; for example, methoxycarbonylamino, ethoxycarbonylamino, t-butoxycarbonylamino, n-octadecyloxycarbonylamino, and N-methyl-methoxycarbonylamino), an aryloxycarbonylamino group (preferably, a substituted or unsubstituted aryloxycarbonylamino group having 7 to 30 carbon atoms; for example, phenoxycarbonylamino, p-chlorophenoxycarbonylamino, and m-n-octyloxyphenoxycarbonylamino), a sulfamoylamino group (preferably, a substituted or unsubstituted sulfamoylamino group having 0 to 30 carbon atoms; for example, sulfamoylamino, N,N-dimethylaminosulfonylamino, and N-n-octylaminosulfonylamino), an alkylsulfonylamino group and an arylsulfonylamino group (preferably, a substituted or unsubstituted alkylsulfonylamino group having 1 to 30 carbon atoms and a substituted or unsubstituted arylsulfonylamino group having 6 to 30 carbon atoms; for example, methylsulfonylamino, butylsulfonylamino, phenylsulfonylamino, 2,3,5-trichlorophenylsulfonylamino, and p-methylphenylsulfonylamino), a mercapto group, an alkylthio group (preferably, a substituted or unsubstituted alkylthio group having 1 to 30 carbon atoms; for example, methylthio, ethylthio, and n-hexadecylthio), an arylthio group (preferably, a substituted or unsubstituted arylthio group having 6 to 30 carbon atoms; for example, phenylthio, p-chlorophenylthio, and m-methoxyphenylthio), a heterocyclic thio group (preferably, a substituted or unsubstituted heterocyclic thio group having 2 to 30 carbon atoms; for example, 2-benzothiazolylthio and 1-phenyltetrazol-5-ylthio), a sulfamoyl group (preferably, a substituted or unsubstituted sulfamoyl group having 0 to 30 carbon atoms; for example, N-ethylsulfamoyl, N-(3-dodecyloxypropyl)sulfamoyl, N,N-dimethylsulfamoyl, N-acetylsulfamoyl, N-benzoylsulfamoyl, and N-(N'-phenylcarbamoyl)sulfamoyl), a sulfo group, an alkylsulfinyl group and an arylsulfinyl group (preferably, a substituted or unsubstituted alkylsulfinyl group having 1 to 30 carbon atoms and a substituted or unsubstituted arylsulfinyl group having 6 to 30 carbon atoms; for example, methylsulfinyl, ethylsulfinyl, phenylsulfinyl, and p-methylphenylsulfinyl), an alkylsulfonyl group and an arylsulfonyl group (preferably, a substituted or unsubstituted alkylsulfonyl group having 1 to 30 carbon atoms and a substituted or unsubstituted arylsulfonyl group having 6 to 30 carbon atoms; for example, methylsulfonyl, ethylsulfonyl, phenylsulfonyl, and p-methylphenylsulfonyl), an acyl group (preferably, a formyl group, a substituted or unsubstituted alkylcarbonyl group having 2 to 30 carbon atoms, and a substituted or unsubstituted arylcarbonyl group having 7 to 30 carbon atoms; for example, acetyl, pivaloyl, 2-chloroacetyl, stearoyl, benzoyl, and p-n-octyloxyphenylcarbonyl), an aryloxycarbonyl group (preferably, a substituted or unsubstituted aryloxycarbonyl group having 7 to 30 carbon atoms; for example, phenoxycarbonyl, o-chlorophenoxycarbonyl, m-nitrophenoxycarbonyl, and p-t-butylphenoxycarbonyl), an alkoxycarbonyl group (preferably, a substituted or unsubstituted alkoxycarbonyl group having 2 to 30 carbon atoms; for example, methoxycarbonyl, ethoxycarbonyl, t-butoxycarbonyl, and n-octadecyloxycarbonyl), a carbamoyl group (preferably, a substituted or unsubstituted carbamoyl group having 1 to 30 carbon atoms; for example, carbamoyl, N-methylcarbamoyl, N,N-dimethylcarbamoyl, N,N-di-n-octylcarbamoyl, and N-(methylsulfonyl)carbamoyl), an arylazo group and a heterocyclic azo group (preferably, a substituted or unsubstituted arylazo group having 6 to 30 carbon atoms and a substituted or unsubstituted heterocyclic azo group having 3 to 30 carbon atoms; for example, phenylazo, p-chlorophenylazo, and 5-ethylthio-1,3,4-thiadiazol-2-ylazo), an imido group (preferably, N-succinimide and N-phthalimide), a phosphino group (preferably, a substituted or unsubstituted phosphino group having 2 to 30 carbon atoms; for example, dimethylphosphino, diphenylphosphino, and methylphenoxyphosphino), a phosphinyl group (preferably, a substituted or unsubstituted phosphinyl group having 2 to 30 carbon atoms; for example, phosphinyl, dioctyloxyphosphinyl, and diethoxyphosphinyl), a phosphinyloxy group (preferably, a substituted or unsubstituted phosphinyloxy group having 2 to 30 carbon atoms; for example, diphenoxyphosphinyloxy and dioctyloxyphosphinyloxy), a phosphinylamino group (preferably, a substituted or unsubstituted phosphinylamino group having 2 to 30 carbon atoms; for example, dimethoxyphosphinylamino and dimethylaminophosphinylamino), a silyl group (preferably, a substituted or unsubstituted silyl group having 3 to 30 carbon atoms; for example, trimethylsilyl, t-butyldimethylsilyl, and phenyldimethylsilyl) are described.

In the case where the group represented by R₁ to R₄ is a group capable of being further substituted, the group represented by R₁ to R₄ may further have a substituent, and in that case, preferable substituent is the group having the same meaning as the substituent described in the explanation of R₁ to R₄. When the group represented by R₁ to R₄ is substituted by two or more substituents, these substituents may be the same or different.

R₅ and R₆ each independently represent an alkyl group, an aryl group, a heterocyclic group, an acyl group, an alkylsulfonyl group, or an arylsulfonyl group. Preferable ranges of the alkyl group, aryl group, heterocyclic group, acyl group, alkylsulfonyl group, or arylsulfonyl group represent the groups having the same meaning as the alkyl group, aryl group, heterocyclic group, acyl group, alkylsulfonyl group, or arylsulfonyl group, which are described above in the explanation of the substituent represented by R₁ to R₄. When the group represented by R₅ or R₆ is a group capable of being further substituted, the group represented by R₅ or R₆ may further have a substituent, and in that case, preferable substituent represents the group having the same meaning as the substituent described in the explanation of R₁ to R₄. When the group represented by R₅ or R₆ is substituted by two or more substituents, these substituents may be the same or different.

R₁ and R₂, R₃ and R₄, R₅ and R₆, R₂ and R₅, or/and R₄ and R₆ may bond to each other in each combination to form a 5-, 6-, or 7-membered ring.

R₇ in formula (I) represents a hydrogen atom, R₁₁-O-CO-, R₁₂-CO-CO-, R₁₃-NH-CO-, R₁₄-SO₂-, R₁₅-W-C(R₁₆)(R₁₇)(R₁₈)-, R₁₉-SO₂NHCO-, R₂₀-CONHCO-, R₂₁-SO₂NHSO₂-, R₂₂-CONHSO₂-, or (M)_{1/n}OSO₂-, wherein R₁₁, R₁₂, R₁₃, R₁₄, R₁₉, R₂₀, R₂₁, and R₂₂ each independently represent an alkyl group, an aryl group, or a heterocyclic group. R₁₅ represents a hydrogen atom or a block group, W represents an oxygen atom, a sulfur atom, or -N(R₁₈)-, and R₁₆, R₁₇ and R₁₈ represent a hydrogen atom or an alkyl group. The alkyl group, aryl group and heterocyclic group represented by R₁₁, R₁₂, R₁₃, R₁₄, R₁₉, R₂₀, R₂₁, or R₂₂ represent the groups having the same meaning as the alkyl group, aryl group and heterocyclic group, which are described above in the explanation of the substituent represented by R₁ to R₄. M represents a cation having a valency of n. In the case where the group represented by R₁₁, R₁₂, R₁₃, R₁₄, R₁₉, R₂₀, R₂₁, or R₂₂ is a group capable of being further substituted, the group represented by R₁₁, R₁₂, R₁₃, R₁₄, R₁₉, R₂₀, R₂₁, or R₂₂ may further have a substituent, and in that case, preferable substituent represents the group having the same meaning as the substituent described in the explanation of R₁ to R₄. When the group represented by R₁₁, R₁₂, R₁₃, R₁₄, R₁₉, R₂₀, R₂₁, or R₂₂ is substituted by two or more substituents, these substituents may be the same or different.

When R₁₆, R₁₇ and R₁₈ represent an alkyl group, R₁₆, R₁₇ and R₁₈ represent the group having the same meaning as the alkyl group described in the explanation of the substituent represented by R₁ to R₄. In the case of where R₁₅ represents a block group, the block group has the same meaning as the block group represented by BLK, which is described below.

Preferable range of the compound represented by formula (I) is explained below. R₁ to R₄ are preferably a hydrogen atom, a halogen atom, an alkyl group, an aryl group, an acylamino group, an alkylsulfonylamino group, an arylsulfonylamino group, an alkoxy group, an aryloxy group, an alkylthio group, an arylthio group, an acyl group, an alkoxycarbonyl group, an aryloxycarbonyl group, a carbamoyl group, a cyano group, a hydroxy group, a carboxy group, a sulfo group, a nitro group, a sulfamoyl group, an alkylsulfonyl group, an arylsulfonyl group, or an acyloxy group, and more preferably a hydrogen atom, a halogen atom, an alkyl group, an acylamino group, an alkylsulfonylamino group, an arylsulfonylamino group, an alkoxy group, an alkylthio group, an arylthio group, an alkoxycarbonyl group, a carbamoyl group, a cyano group, a hydroxy group, a carboxy group, a sulfo group, a nitro group, a sulfamoyl group, an alkylsulfonyl group, or an arylsulfonyl group. It is particularly preferable that, among R₁ to R₄, one of R₁ or R₃ is a hydrogen atom.

R₅ and R₆ are preferably an alkyl group, an aryl group, or a heterocyclic group, and most preferably an alkyl group.

It is preferred from the viewpoint of being compatible in coloring property and storability that the oxidization potential of p-phenylenediamine derivative, in which R₇ of the compound represented by formula (I) is a hydrogen atom, is 5 mV or less (with respect to SCE) in an aqueous solution having the pH of 10.

R₇ is preferably a hydrogen atom, R₁₁-O-CO-, R₁₄-SO₂-, R₁₉-SO₂-NH-CO-, or R₁₅-W-C(R₁₆)(R₁₇)(R₁₈)-, more preferably a hydrogen atom, R₁₁-O-CO-, or R₁₉-SO₂-NH-CO-, and most preferably a hydrogen atom or R₁₉-SO₂-NH-CO-. R₁₁ is preferably an alkyl group, and R₁₁ is preferably a group containing a timing group that causes a cleavage reaction using an electron transfer reaction, which is described in U.S. Patent (USP) Nos. 4,409,323 and 4,421,845, and R₁₁ is preferably a group represented by the following formula (T-1), in which the terminal which causes the electron transfer reaction of the timing group is blocked.

Formula (T-1) BLK-W-(X=Y)ⱼ-C(R₂₁)R₂₂-**

In the formula, BLK represents a block group, ** denotes a bond with -O-CO- at this position, W represents an oxygen atom, a sulfur atom, or -N(R₂₃)-, X and Y each represent a methine or a nitrogen atom, j represents 0, 1, or 2, and R₂₁, R₂₂ and R₂₃ each represent a hydrogen atom or the group having the same meaning as the substituent explained in R₁ to R₄. Here, when X and Y represent a substituted methine, it may be any of the case in which the substituent and two arbitrary substituents of R₂₁, R₂₂, and R₂₃ bond together to form a cyclic structure (for example, a benzene ring or a pyrazole ring) or the case in which a cyclic structure is not formed.

As a block group represented by BLK, known compounds can be used. Namely, a block group such as an acyl group, a sulfonyl group, and the like described in Japanese Patent Application Publication (JP-B) No. 48-9968, JP-A Nos. 52-8828, 57-82834, USP No. 3,311,476, JP-B No. 47-44805 (USP No. 3,615,617), and the like; a block group utilizing a reverse Michael reaction described in JP-B Nos. 55-17369 (USP No. 3,888,677), 55-9696 (USP No. 3,791,830), 55-34927 (USP No. 4,009,029), JP-A Nos. 56-77842 (USP No. 4,307,175), 59-105640, 59-105641, and 59-105642, and the like; a block group utilizing formation of quinonemethide or quinonemethide-like compound by an intramolecular electron transfer described in JP-B No. 54-39727, USP Nos. 3,674,478, 3,932,480, 3,993,661, JP-A Nos. 57-135944, 57-135945 (USP No. 4,420,554), 57-136640, 61-196239, 61-196240 (USP No. 4,702,999), 61-185743, 61-124941 (USP No. 4,639,408), JP-A No. 2-280140 and the like; a block group utilizing an intramolecular nucleophilic substitution reaction described in USP Nos. 4,358,525 and 4,330,617, JP-A Nos. 55-53330 (USP No. 4,310,612), 59-121328, 59-218439, and 63-318555 (European Patent Application Laid-Open (EP-A) No. 0295729), and the like; a block group utilizing a ring cleavage reaction of 5- or 6-membered ring described in JP-A Nos. 57-76541 (USP No. 4,335,200), 57-135949 (USP No. 4,350,752), 57-179842, 59-137945, 59-140445, 59-219741, 59-202459, 60-41034 (USP No. 4,618,563), 62-59945 (USP No. 4,888,268), 62-65039 (USP No. 4,772,537), 62-80647, 3-236047, and 3-238445 and the like; a block group utilizing an addition reaction of a nucleophile to a conjugated unsaturated bond described in JP-A Nos. 59-201057 (USP No. 4,518,685), 61-43739 (USP No. 4,659,651), 61-95346 (USP No. 4,690,885), and 61-95347 (USP No. 4,892,811), JP-A Nos. 64-7035, 4-42650 (USP No. 5,066,573), 1-245255, 2-207249, 2-235055 (USP No. 5,118,596), and 4-186344 and the like; a block group utilizing a β-elimination reaction described in JP-A Nos. 59-93442, 61-32839, and 62-163051, JP-B No. 5-37299, and the like; a block group utilizing a nucleophilic substitution reaction of diarylmethanes described in JP-A No. 61-188540; a block group utilizing the Rossen's transition reaction described in JP-A No. 62-187850; a block group utilizing the reaction of N-acyl compound of thiazolidine-2-thione and amines described in JP-A Nos. 62-80646, 62-144163, and 62-147457 and the like; a block group, which has two electrophilic groups and reacts with a dinucleophilic agent, described in JP-A Nos. 2-296240 (USP No. 5,019,492), 4-177243, 4-177244, 4-177245, 4-177246, 4-177247 4-177248, 4-177249, 4-179948, 4-184337, and 4-184338, International Patent Publication (WO) No. 92/21064, JP-A No. 4-330438, WO No. 93/03419, JP-A No. 5-45816, and the like; and a block group described in JP-A Nos. 3-236047 and 3-238445 can be described. Among these block groups, the block group having two electrophilic groups which reacts with a dinucleophilic agent, described in JP-A Nos. 2-296240 (USP No. 5,019,492), 4-177243, 4-177244, 4-177245, 4-177246, 4-177247, 4-177248, 4-177249, 4-179948, 4-184337, and 4-184338, WO No. 92/21064, JP-A No. 4-330438, WO No. 93/03419, JP-A No. 5-45816, and the like is particularly preferable.

Specific examples of the timing group part excluding BLK from the group represented by formula (T-1) are shown below. In the following, * denotes a bond with BLK at this position and ** denotes a bond with -O-CO- at this position.

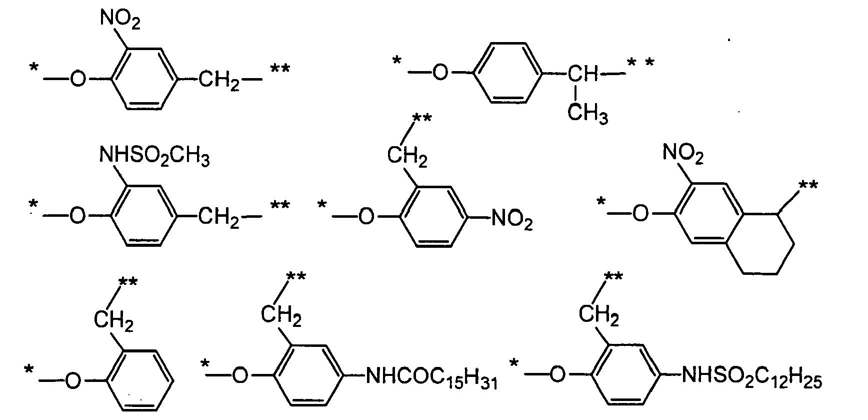

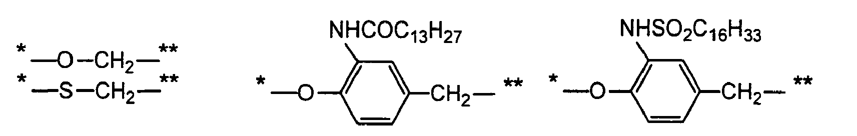

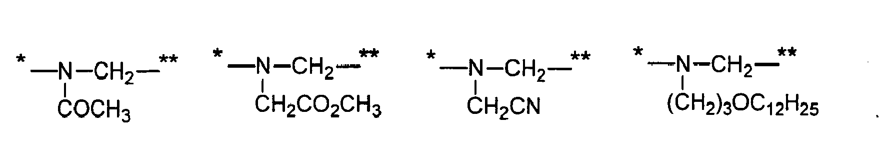

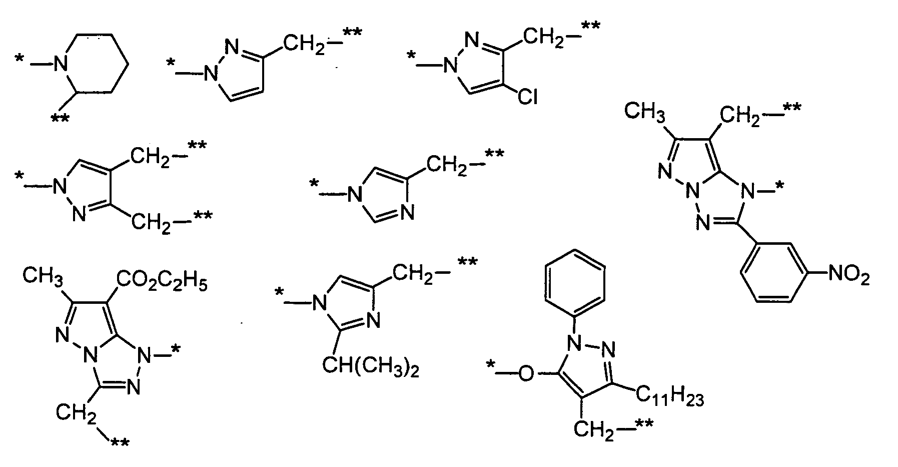

R₁₂ and R₁₃ are preferably an alkyl group or an aryl group, and R₁₄ is preferably an aryl group. R₁₅ is preferably a block group and preferable block groups are the same as those of preferable BLK among the groups represented by the above-mentioned formula (T-1). R₁₆, R₁₇, and R₁₈ are preferably a hydrogen atom.

As the compound represented by formula (I) used in the present invention, the compounds described in USP Nos. 5,242,783 and 4,426,441, and JP-A Nos. 62-227141, 5-257225, 5-249602, 6-43607, and 7-333780 are also preferable.

Specific examples of the compound represented by formula (I) according to the present invention are shown below, but the present invention is not limited these compounds.

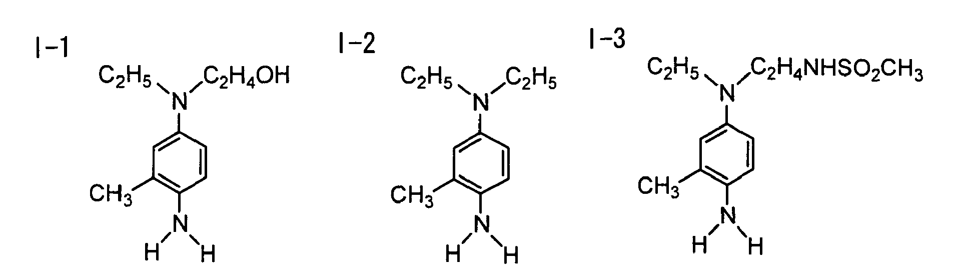

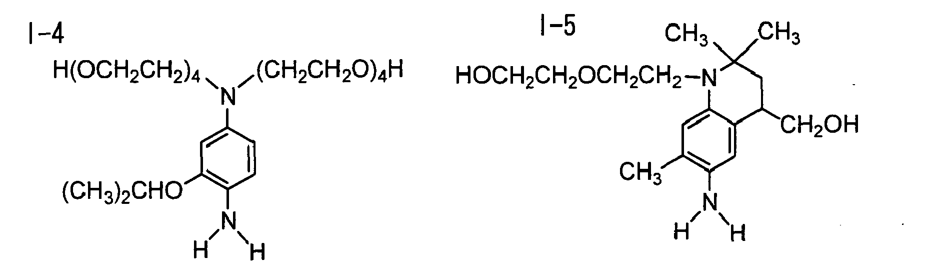

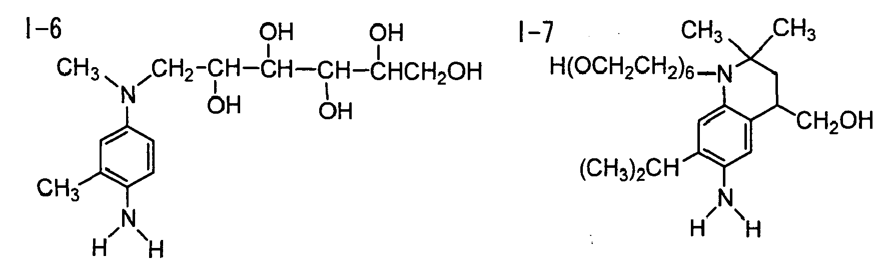

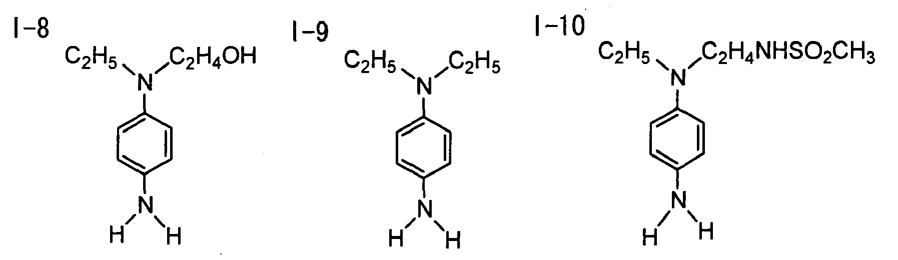

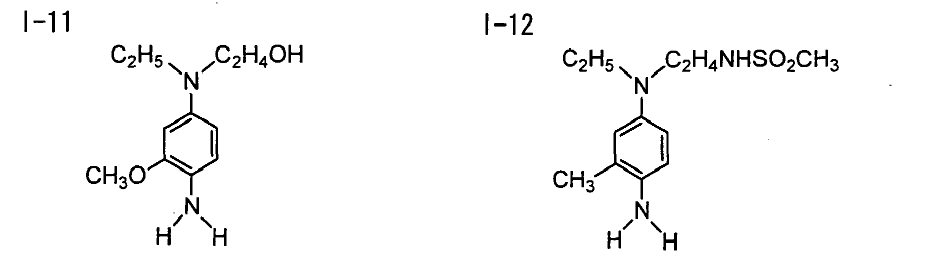

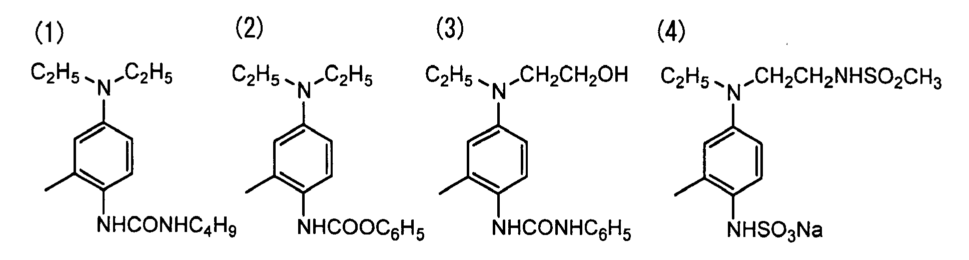

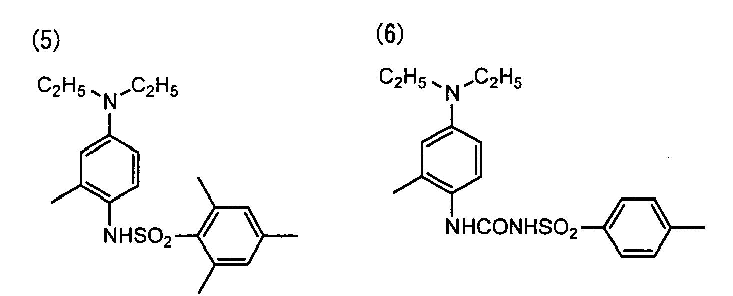

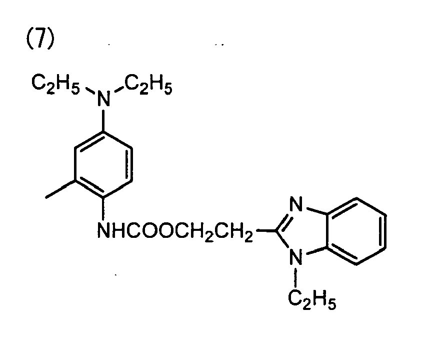

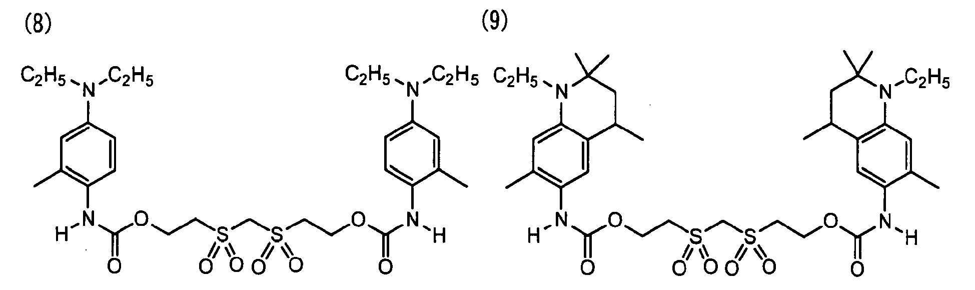

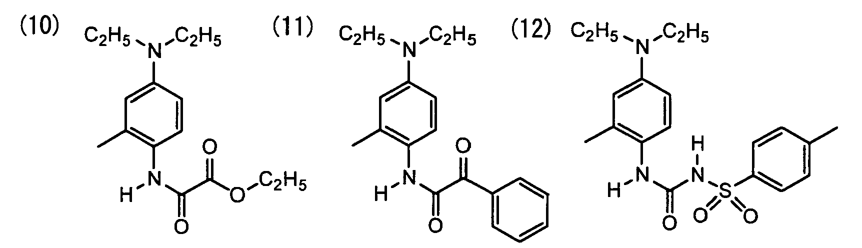

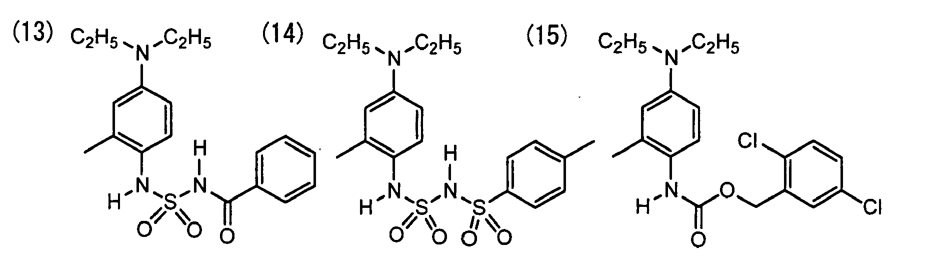

Another preferable embodiment of the color developing agent used in the present invention is a compound represented by the following formula (II).

Formula (II) R¹¹-NH-NH-X-R¹²

In formula (II), R¹¹ represents an aryl group or a heterocyclic group, each of which may have a substituent; R¹² represents an alkyl group, an alkenyl group, an alkynyl group, an aryl group, or a heterocyclic group, each of which may have a substituent; X represents -SO₂-, -CO-, -COCO-, -CO-O-, -CO-N(R¹³)-, -COCO-O-, - COCO-N(R¹³)-, or -SO₂-N(R¹³)-; and R¹³ represents a hydrogen atom or a group selected from the groups that may be represented by R¹².

One of preferable embodiments of the compound represented by formula (II) is a compound represented by the following formula (III).

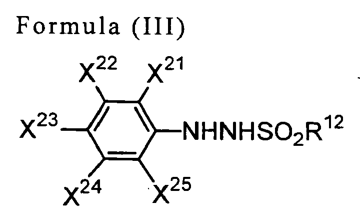

In formula (III), R¹² represents an alkyl group or a heterocyclic group. X²¹, X²³ and X²⁵ each independently represent a hydrogen atom, a nitro group, a cyano group, an alkylsulfonyl group, an arylsulfonyl group, an alkylsulfinyl group, an arylsulfinyl group, a sulfamoyl group, a carbamoyl group, an alkoxycarbonyl group, an aryloxycarbonyl group, an acyl group, or a trifluoromethyl group.
X²² and X²⁴ each independently represent a hydrogen atom, a nitro group, a cyano group, an alkylsulfonyl group, an arylsulfonyl group, an alkylsulfinyl group, an arylsulfinyl group, a sulfamoyl group, a carbamoyl group, an alkoxycarbonyl group, an aryloxycarbonyl group, an acyl group, a trifluoromethyl group, a halogen atom, an acyloxy group, or an acylthio group. However, the sum of Hammett's σp values of X²¹, X²³ and X²⁵ and Hammett's σm values of X²² and X²⁴ is 1.5 or more.

Another preferable embodiment of the compound represented by formula (II) is a compound represented by the following formula (IV).

Formula (IV) R¹¹-NH-NH-CO-R¹²

In formula (IV), R¹¹ and R¹² each have the same meaning as that in formula (II).

An oxidation product of the color developing agent used in the present invention is a compound which is **characterized in that** it reacts with a dye precursor to form a dye.

The structure of the color developing agent is to be described in detail hereinafter. R¹¹ represents an aryl group or a heterocyclic group, each of which may have a substituent. The aryl group represented by R¹¹ is preferably an aryl group having 6 to 14 carbon atoms, and examples thereof include a phenyl group and a naphthyl group. The heterocyclic group represented by R¹¹ is preferably a saturated or unsaturated, 5-, 6- or 7-membered heterocyclic group including at least one atom selected from among nitrogen, oxygen, sulfur, and selenium. Furthermore, they may combine with a benzene ring or a heterocycle to form a condensed ring. Specific examples of the heterocyclic group represented by R¹¹ include furanyl, thienyl, oxazolyl, thiazolyl, imidazolyl, triazolyl, pyrrolidinyl, benzoxazolyl, benzothiazolyl, pyridyl, pyridazyl, pyrimidinyl, pyrazinyl, triazinyl, quinolinyl, isoquinolinyl, phthalazinyl, quinoxalinyl, quinazolinyl, purinyl, pteridinyl, azepinyl, benzoxepinyl and the like.

Examples of the substituent for R¹¹ include an alkyl group, an alkenyl group, an alkynyl group, an aryl group, a heterocyclic group, an alkoxy group, an aryloxy group, a heterocyclic oxy group, an alkylthio group, an arylthio group, a heterocyclic thio group, an acyloxy group, an acylthio group, an alkoxycarbonyloxy group, an aryloxycarbonyloxy group, a carbamoyloxy group, an alkylsulfonyloxy group, an arylsulfonyloxy group, an amino group, an alkylamino group, an arylamino group, an amido group, an alkoxycarbonylamino group, an aryloxycarbonylamino group, a ureido group, a sulfonamido group, a sulfamoylamino group, an acyl group, an alkoxycarbonyl group, an aryloxycarbonyl group, a carbamoyl group, an acylcarbamoyl group, a carbamoylcarbamoyl group, a sulfonylcarbamoyl group, a sulfamoylcarbamoyl group, an alkylsulfonyl group, an arylsulfonyl group, an alkylsulfinyl group, an arylsulfinyl group, an alkoxysulfonyl group, an aryloxysulfonyl group, a sulfamoyl group, an acylsulfamoyl group, a carbamoylsulfamoyl group, a halogen atom, a nitro group, a cyano group, a carboxy group, a sulfo group, a phosphono group, a hydroxy group, a mercapto group, an imido group, an azo group and the like. R¹² represents an alkyl group, an alkenyl group, an alkynyl group, an aryl group, or a heterocyclic group, each of which may have a substituent.

The alkyl group represented by R¹² is preferably a straight chain, branched or cyclic alkyl group having 1 to 16 carbon atoms, and examples thereof include methyl, ethyl, hexyl, dodecyl, 2-octyl, t-butyl, cyclopentyl, cyclooctyl and the like. The alkenyl group represented by R¹² is preferably a chain or cyclic alkenyl group having 2 to 16 carbon atoms, and examples thereof include vinyl, 1-octyl and cyclohexenyl.

The alkynyl group represented by R¹² is preferably an alkynyl group having 2 to 16 carbon atoms, and examples thereof include 1-butynyl, phenylethynyl and the like. Examples of the aryl group and the heterocyclic group represented by R¹² include the same groups as those described for R¹¹. Examples of the substituent for R¹² include the same substituents as those described for R¹¹. Specific examples of X preferably include -SO₂-, -CO-, -COCO- and -CO-N(R¹³)-, and more preferably -SO₂- and -CO-N(R¹³)-. Especially, when X represents -CO-N(R¹³)-, a two-equivalent dye precursor can be used as the dye precursor that reacts with this compound, and therefore, increase in stain during long-term storage of unprocessed protease detection materials can be effectively depressed, which is preferable.

R¹¹ is preferably a nitrogen-containing heterocyclic group or a group represented by the following formula (V). Concerning the compound represented by formula (IV), R¹¹ is preferably a 6-membered nitrogen-containing heterocyclic group or a group represented by formula (V). Concerning the compound represented by formula (II), R¹¹ is preferably a group represented by formula (V).

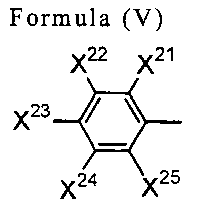

In formula (V), X²¹, X²³ and X²⁵ each independently represent a hydrogen atom, a nitro group, a cyano group, an alkylsulfonyl group, an arylsulfonyl group, an alkylsulfinyl group, an arylsulfinyl group, a sulfamoyl group, a carbamoyl group, an alkoxycarbonyl group, an aryloxycarbonyl group, an acyl group, or a trifluoromethyl group.
X²² and X²⁴ each independently represent a hydrogen atom, a nitro group, a cyano group, an alkylsulfonyl group, an arylsulfonyl group, an alkylsulfinyl group, an arylsulfinyl group, a sulfamoyl group, a carbamoyl group, an alkoxycarbonyl group, an aryloxycarbonyl group, an acyl group, a trifluoromethyl group, a halogen atom, an acyloxy group, or an acylthio group. However, the sum of Hammett's σp values of X²¹, X²³ and X²⁵ and Hammett's σm values of X²² and X²⁴ is 1.5 or more. The color developing agent used in the present invention is preferably non-diffusible in the layer.

Specific examples of the compound represented by formula (II) are shown below, but the invention is not limited to these compounds.

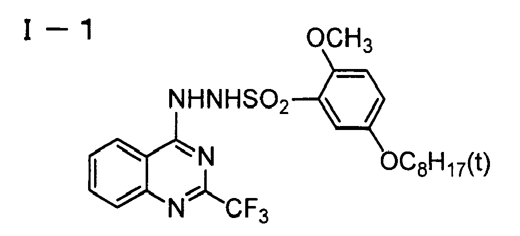

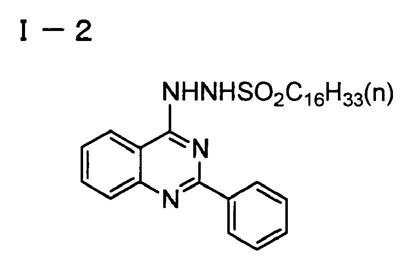

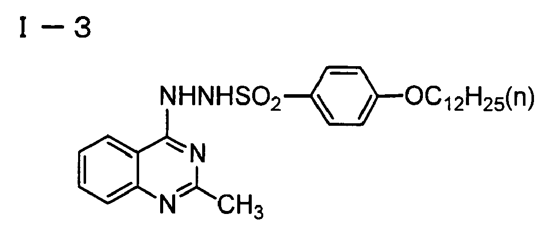

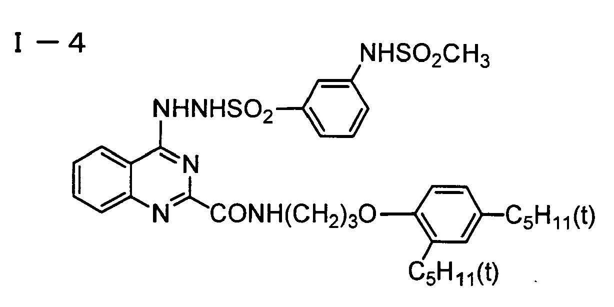

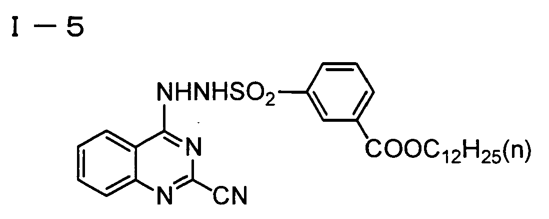

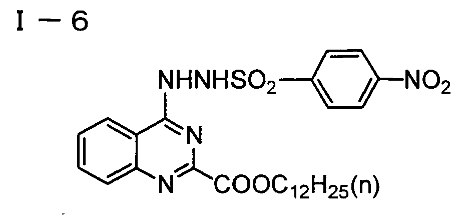

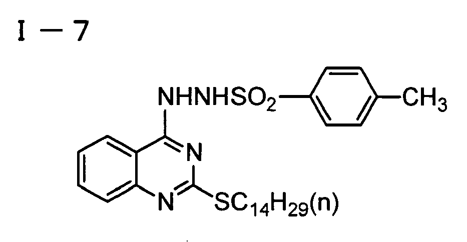

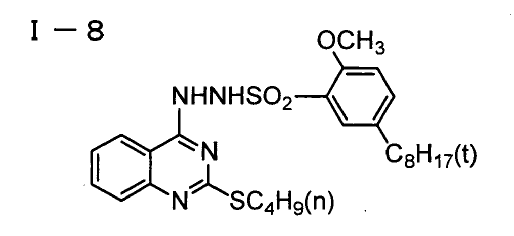

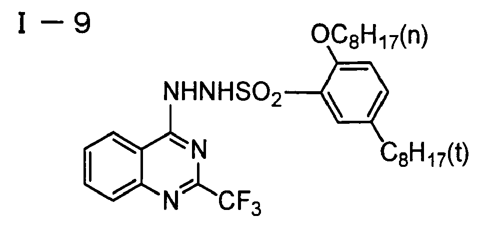

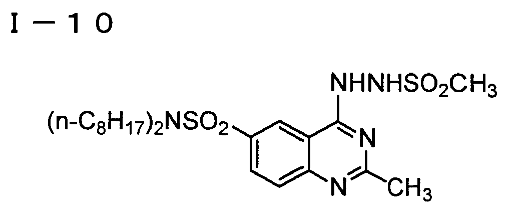

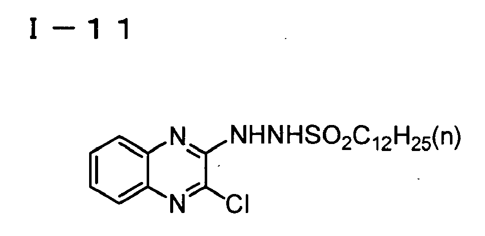

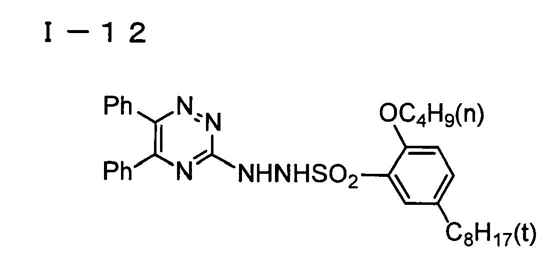

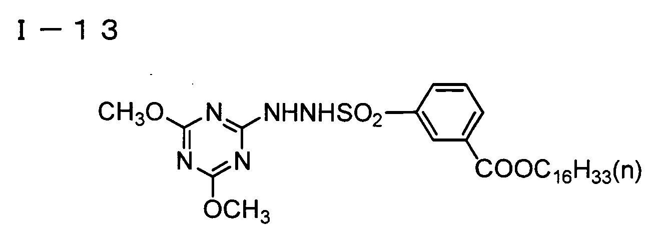

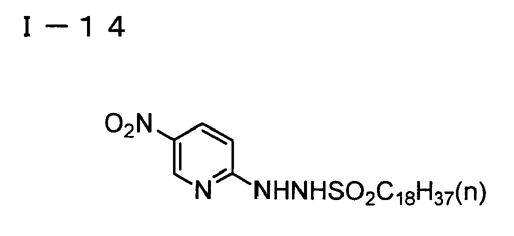

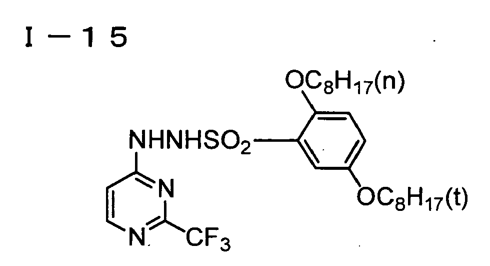

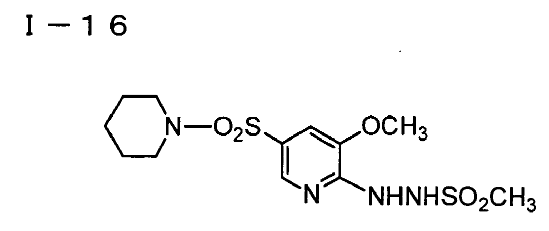

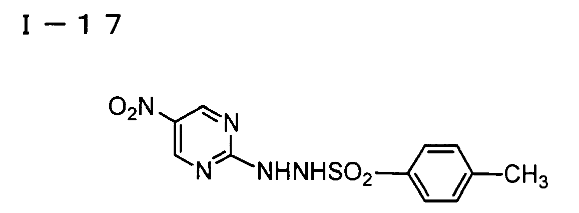

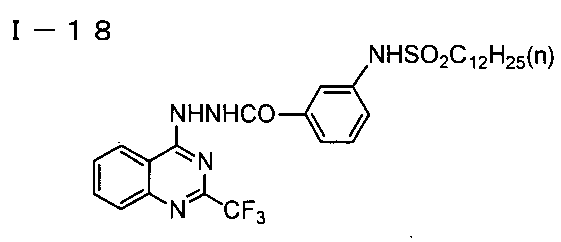

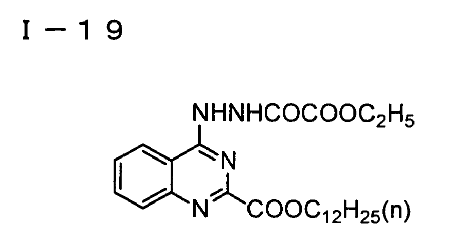

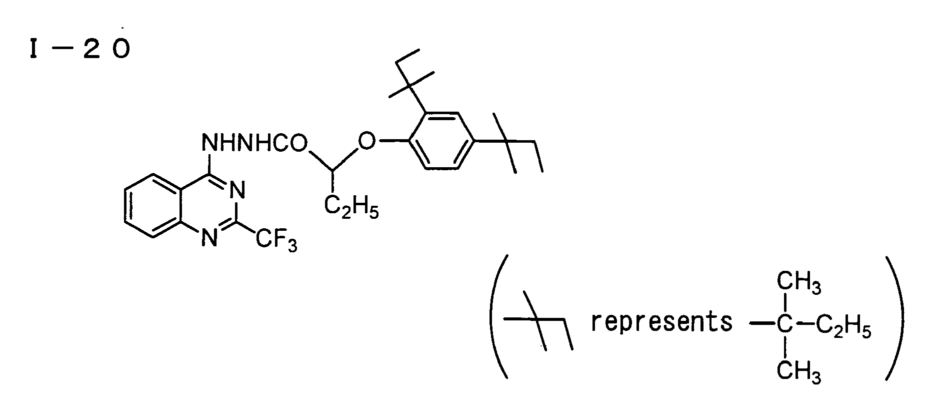

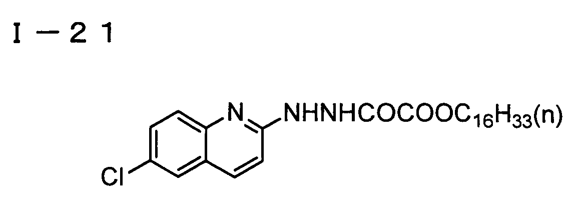

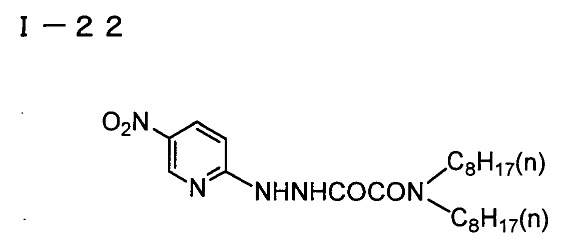

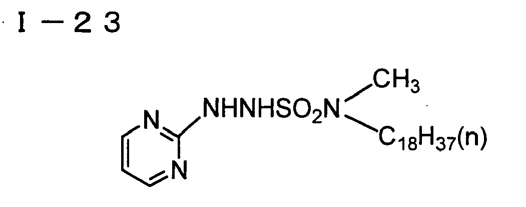

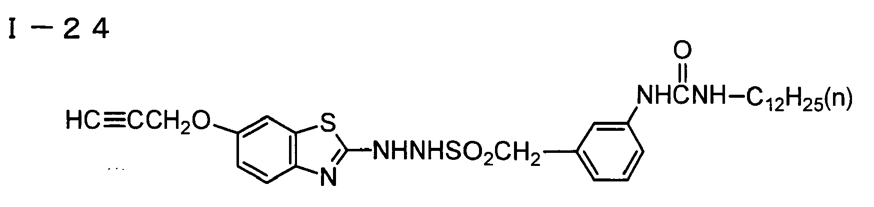

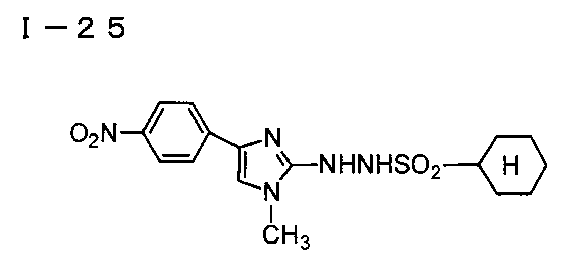

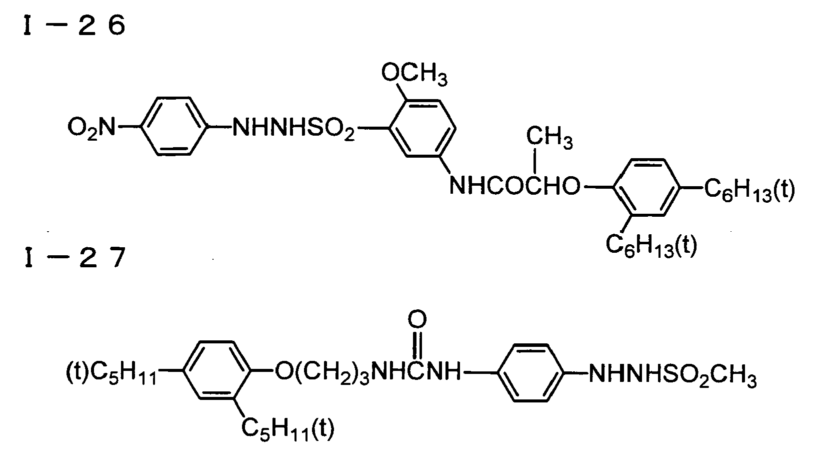

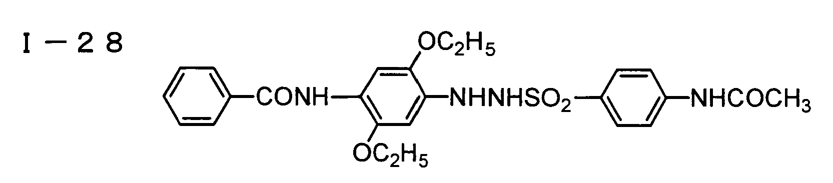

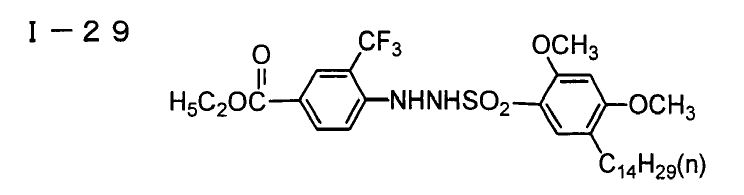

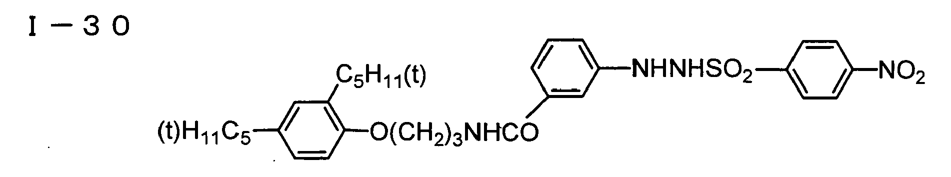

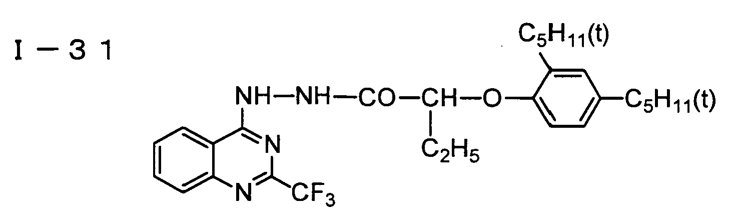

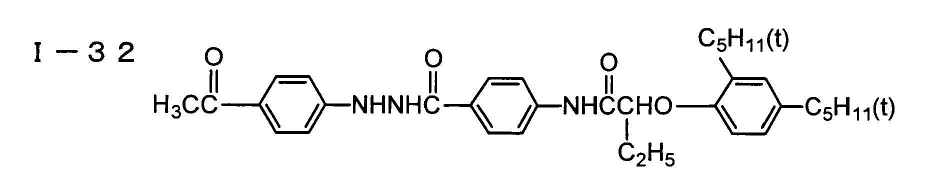

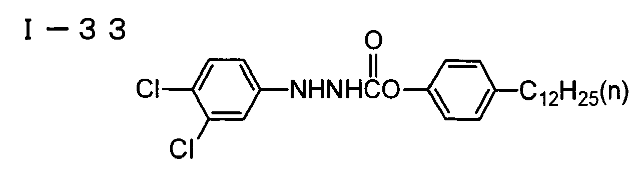

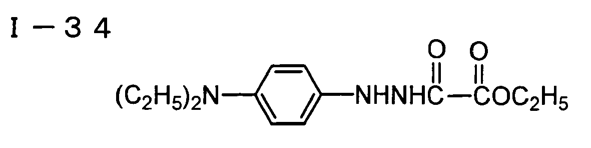

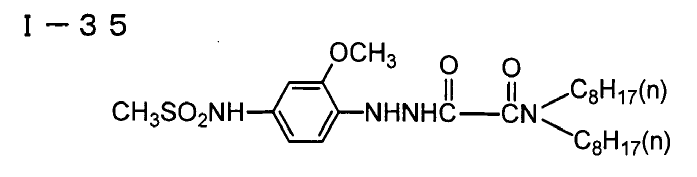

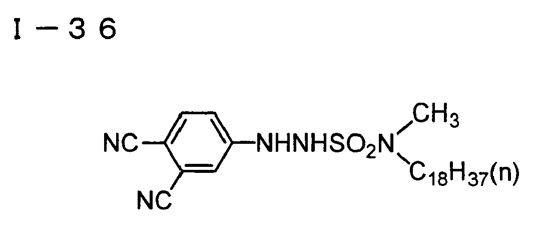

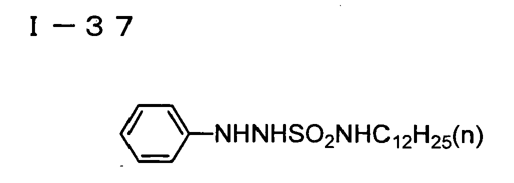

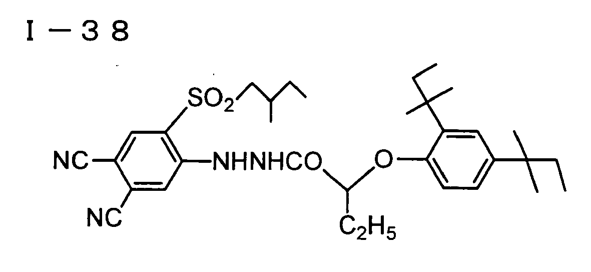

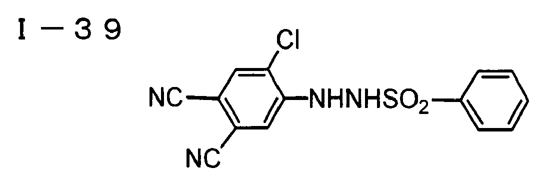

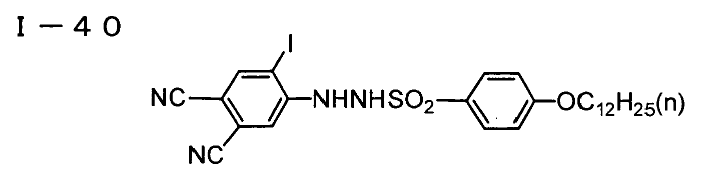

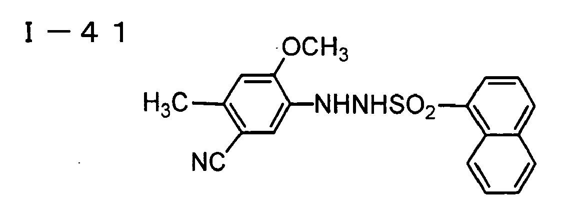

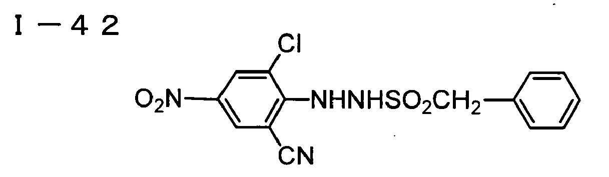

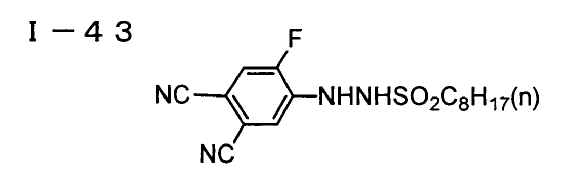

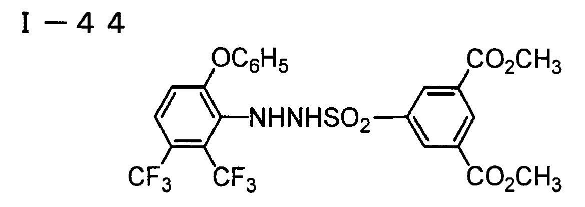

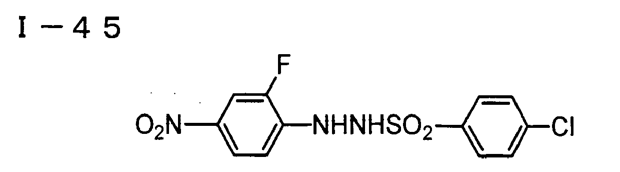

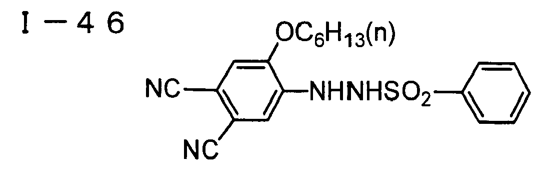

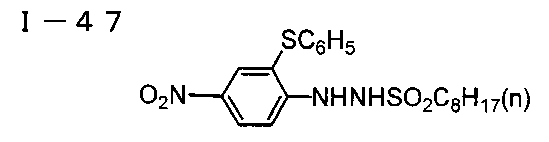

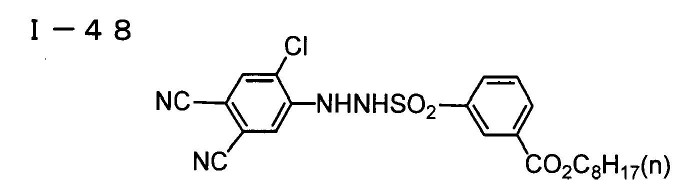

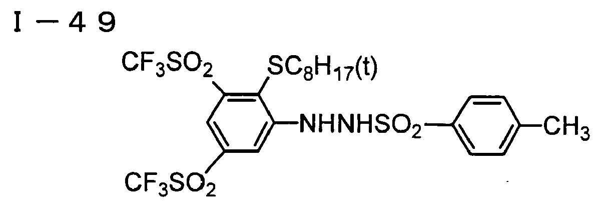

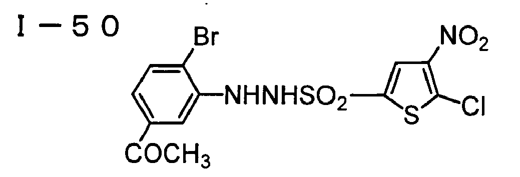

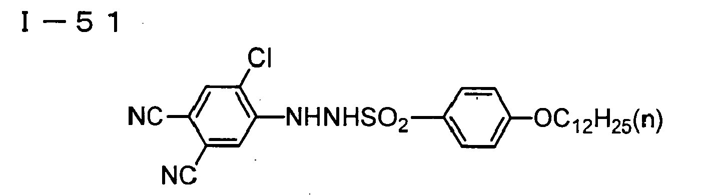

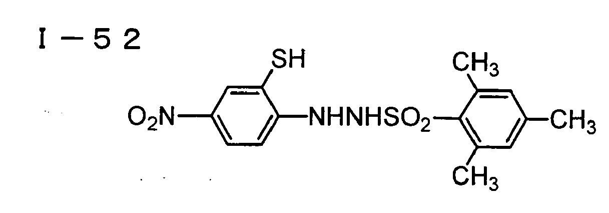

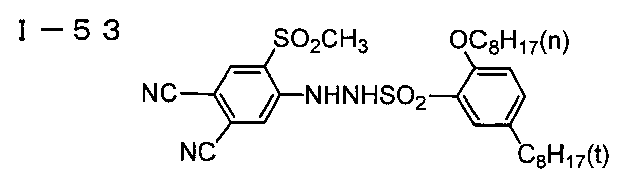

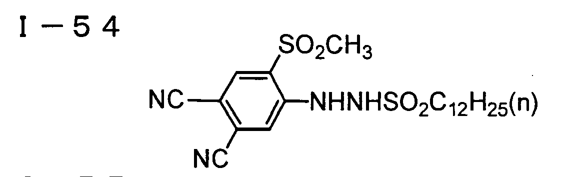

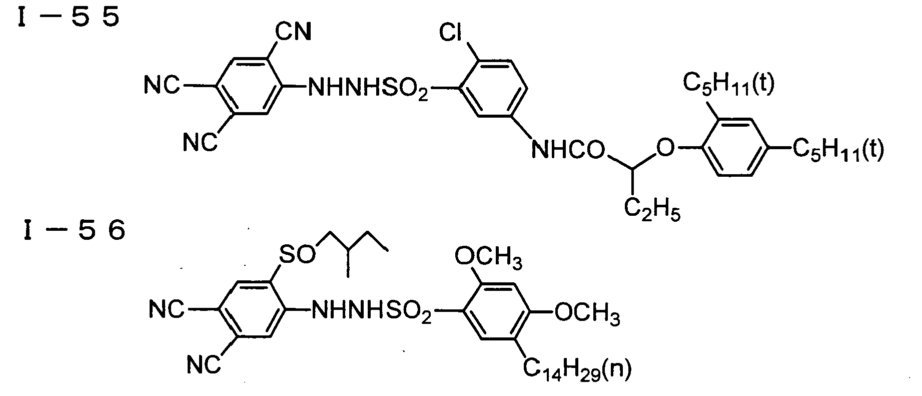

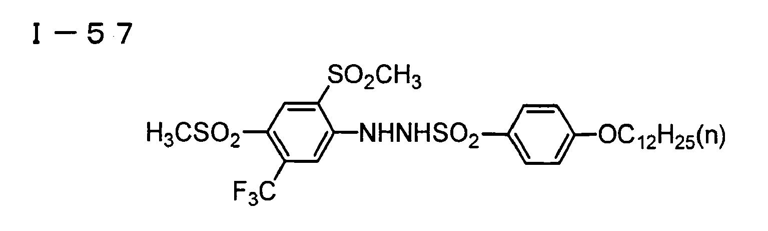

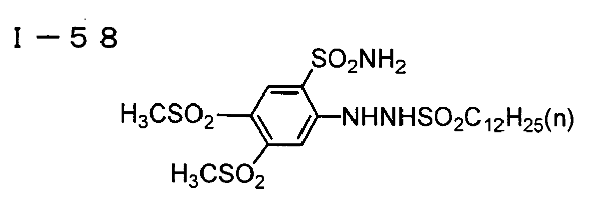

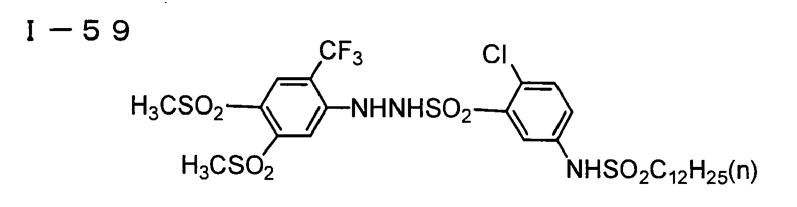

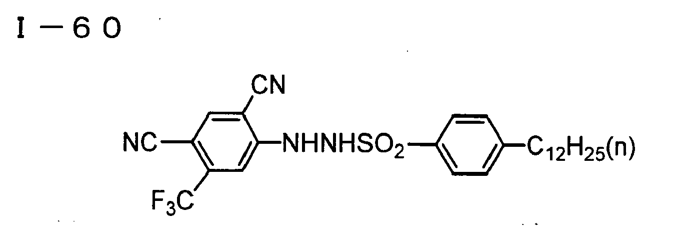

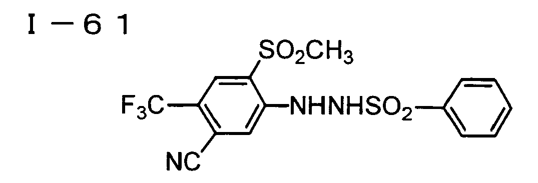

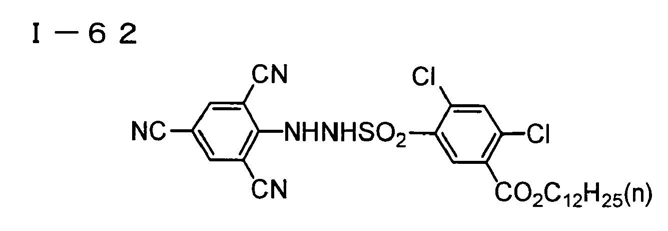

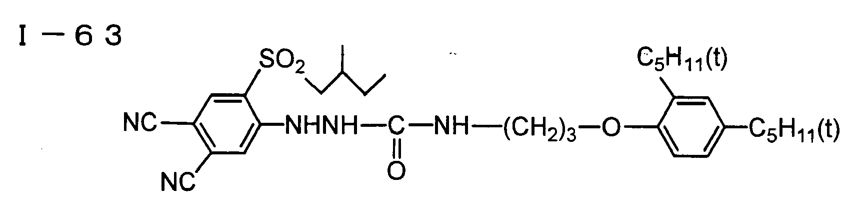

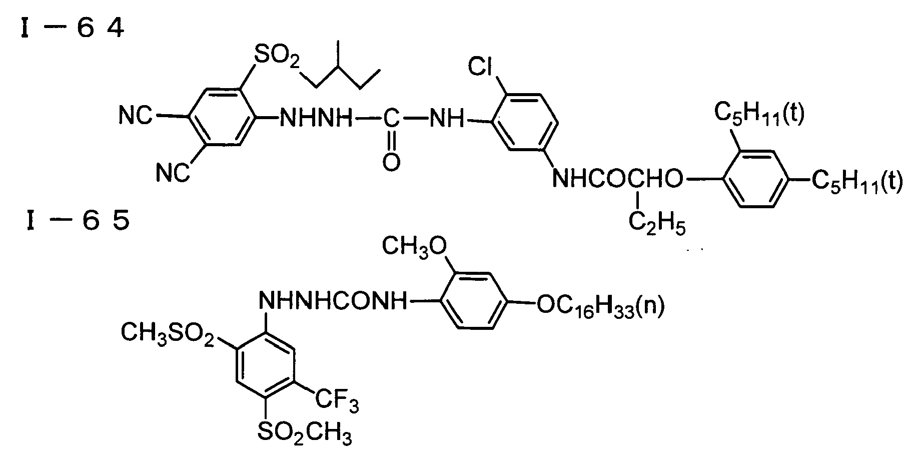

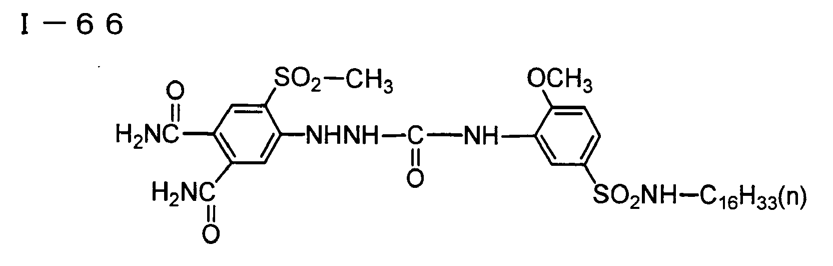

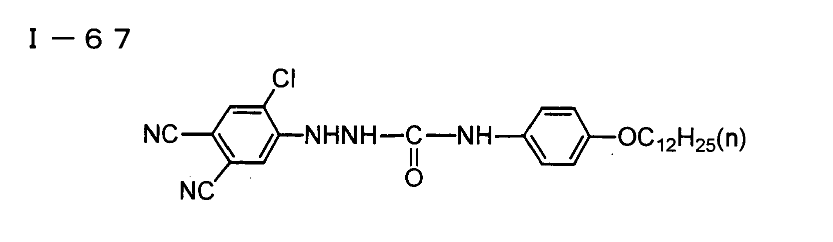

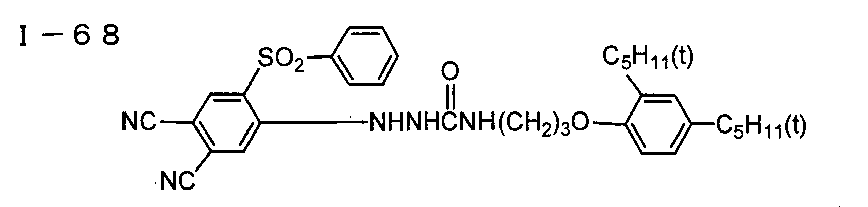

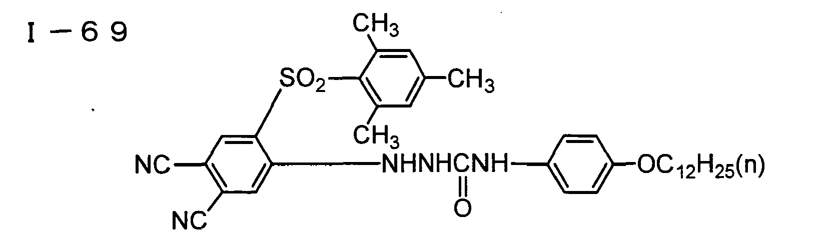

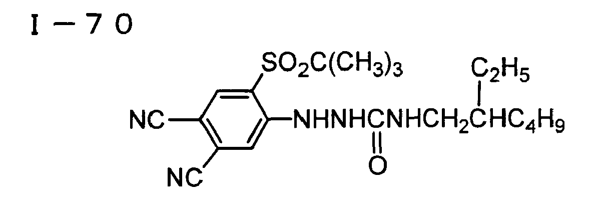

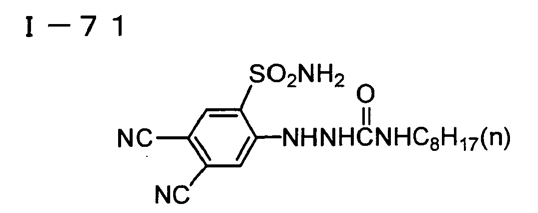

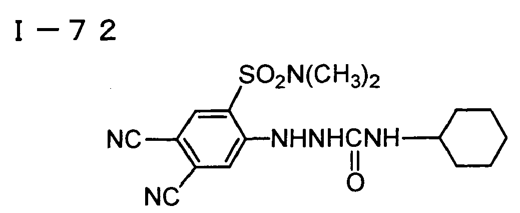

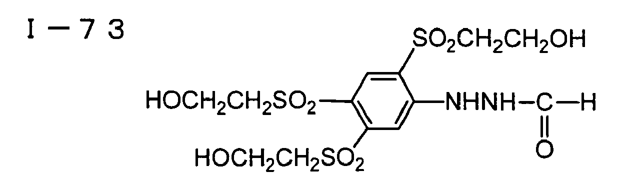

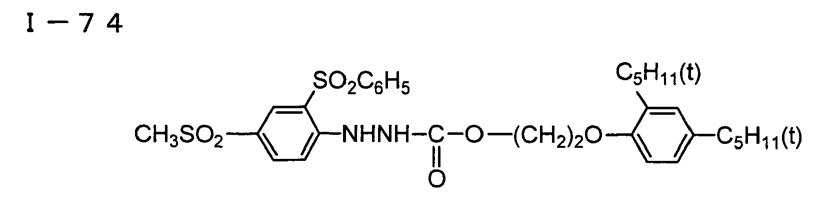

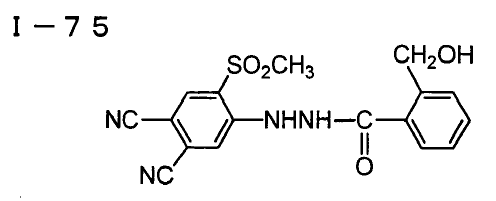

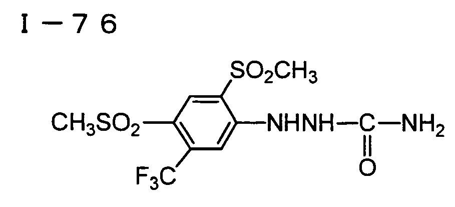

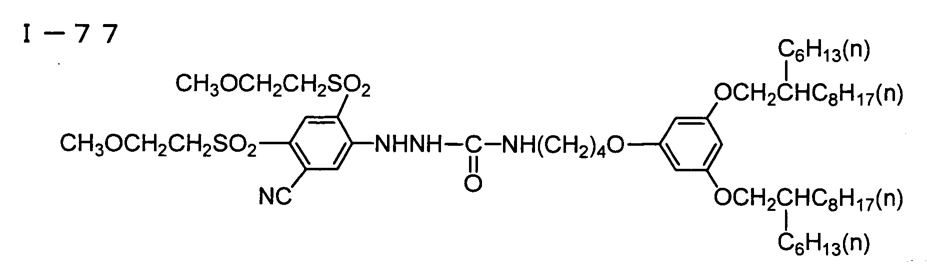

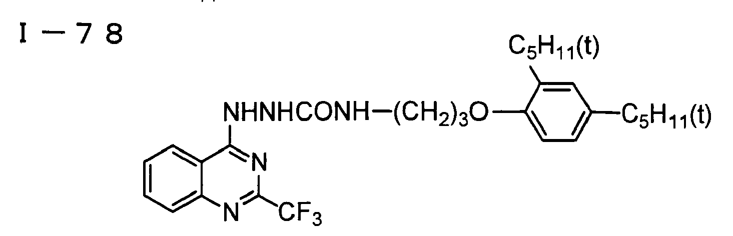

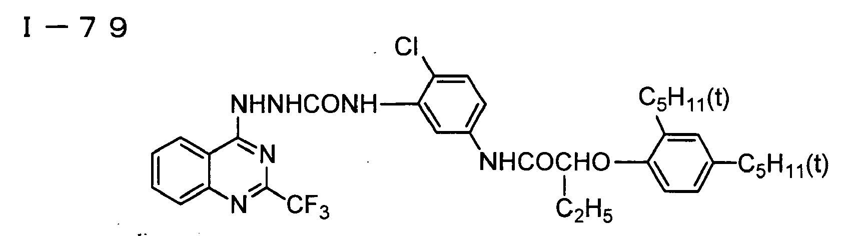

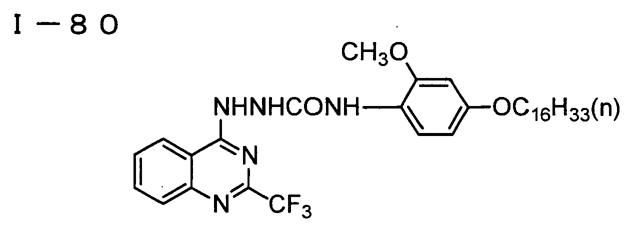

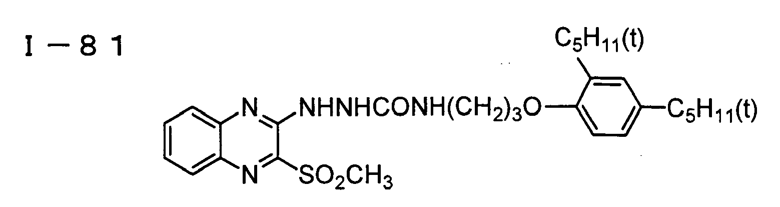

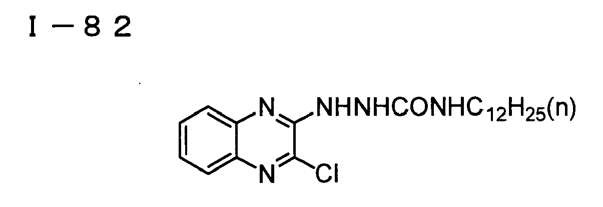

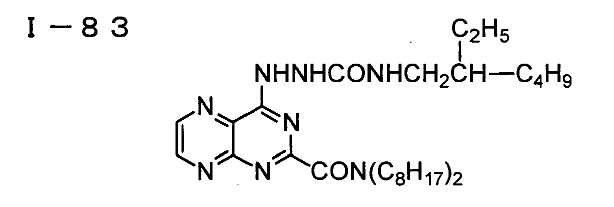

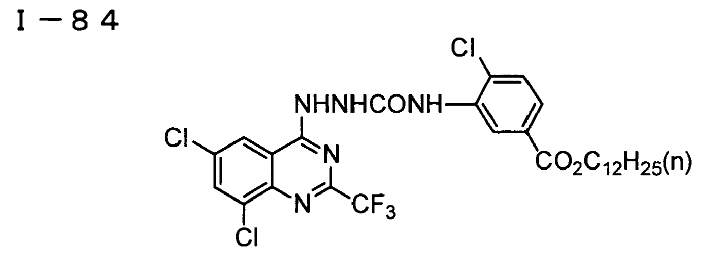

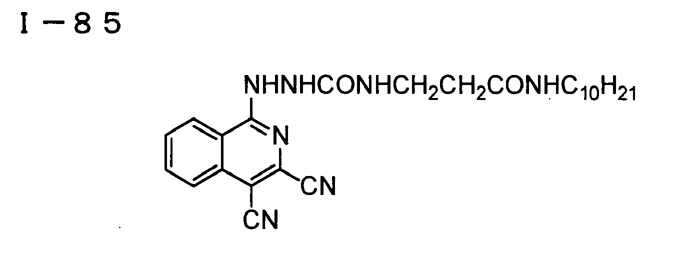

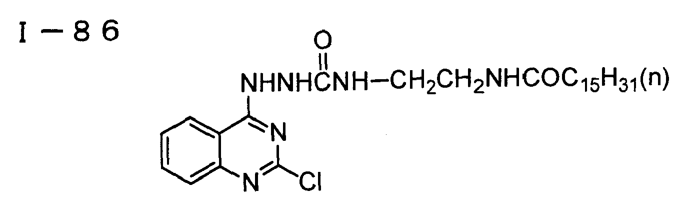

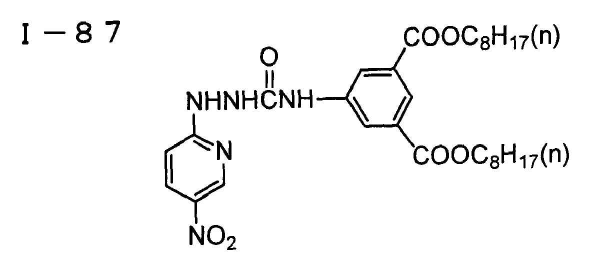

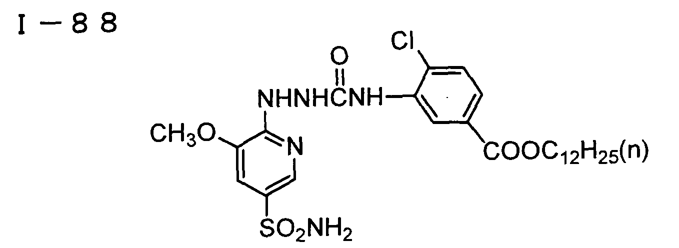

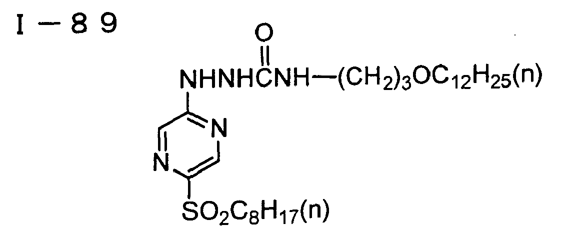

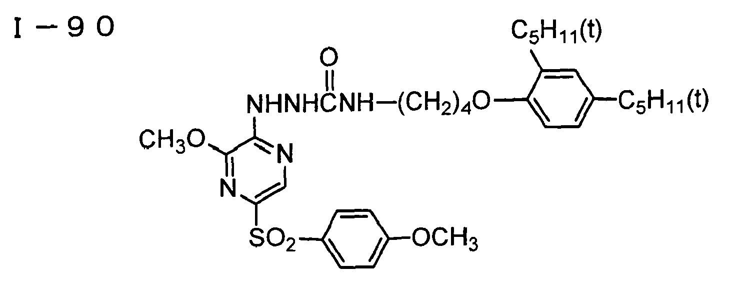

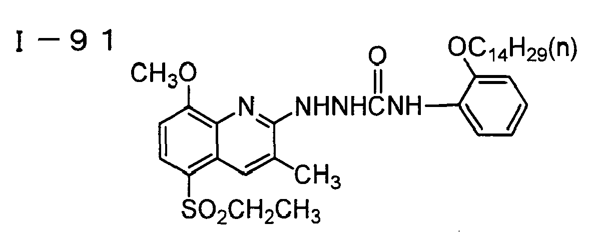

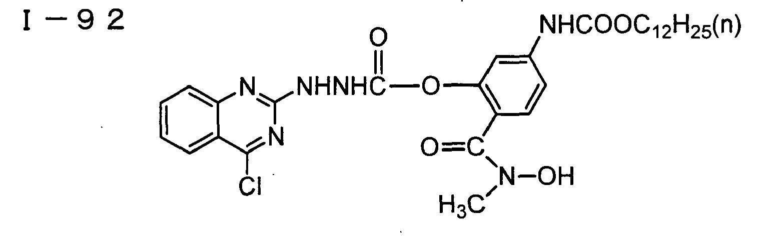

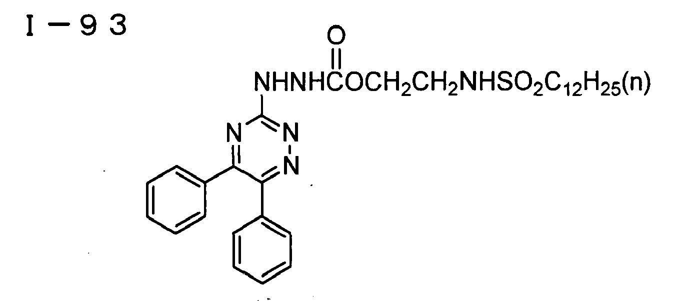

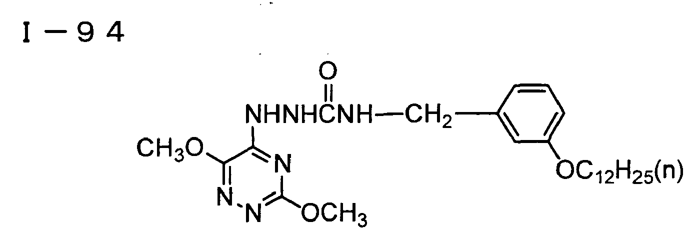

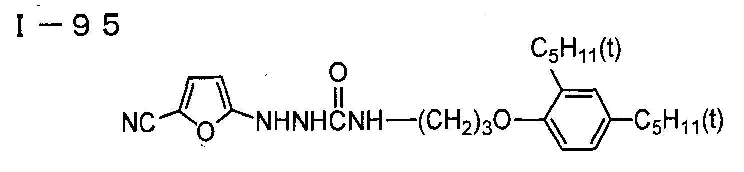

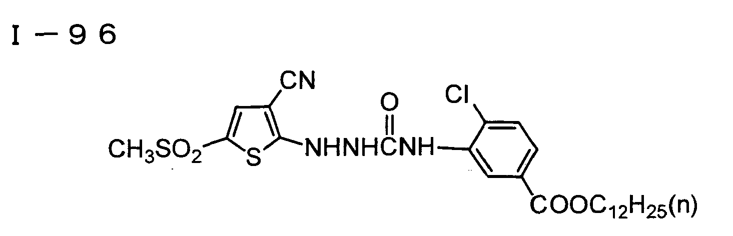

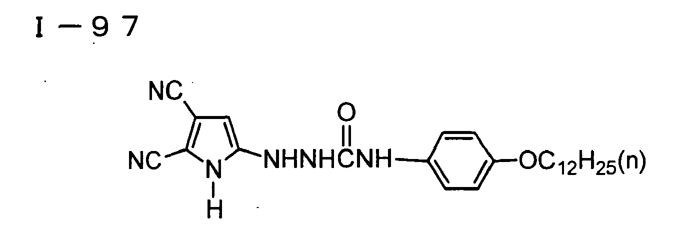

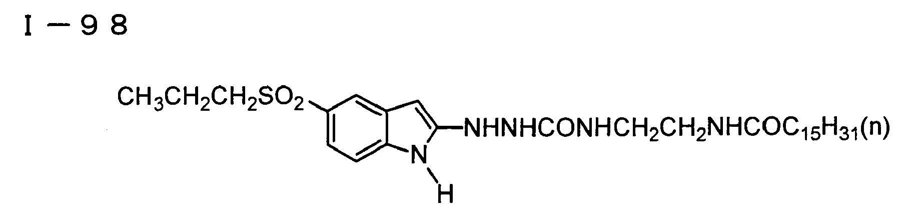

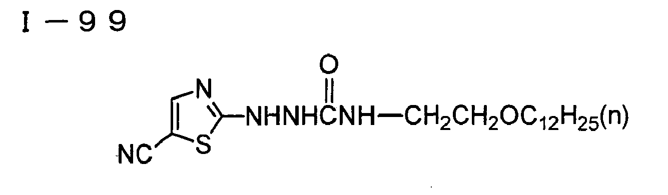

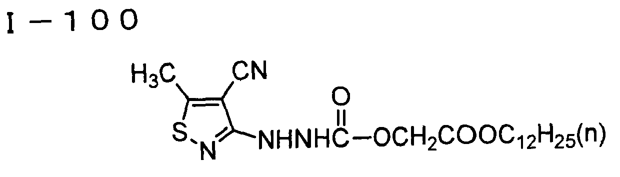

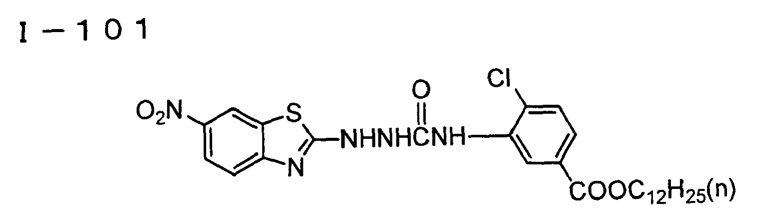

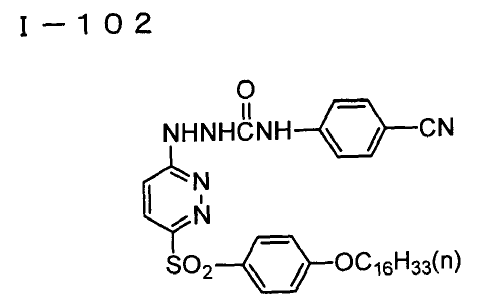

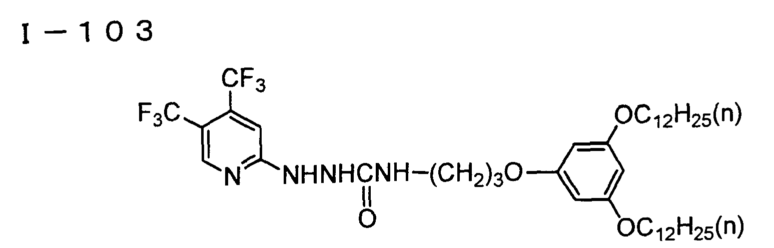

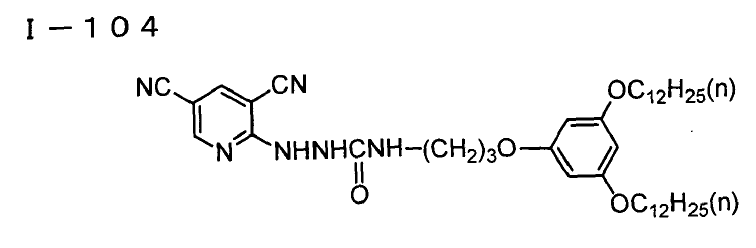

In the present invention, the color developing agent can be used in the form of a solution by dissolving it in an aqueous solution or organic solvent, an emulsified dispersion, or a solid dispersion.

In the case where the color developing agent is used in the form of fine particle dispersion, a number-average particle size of the color developing agent is preferably from 0.001 µm to 5 µm, and more preferably from 0.001 µm to 0.5 µm.

The addition amount of the color developing agent in the present invention is preferably in a range of from equivalent by mole to 1000 times by mole with respect to 1 mol of the dye precursor used, and more preferably in a range of from equivalent by mole to 100 times by mole.

### 3) Dye precursor

The dye precursor used in the present invention is a compound which forms a dye by reacting with an oxidation product of the color developing agent according to the present invention. It is preferably a compound which forms a dye having an absorption in the visible light region by coupling with an oxidation product of the color developing agent. Such a compound is a compound that is well known for the color photographic system, and as representative examples, a pyrrolotriazole type coupler, a phenol type coupler, a naphthol type coupler, a pyrazolotriazole type coupler, a pyrazolone type coupler, an acylacetoanilide type coupler, and the like are described.

In the present invention, preferable dye precursor is a dye precursor having the structure represented by formula (C-1), (C-2), (C-3), (M-1), (M-2), (M-3), (Y-1), (Y-2), or (Y-3):

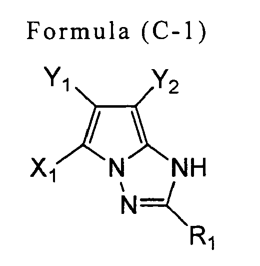

(wherein X₁ represents a hydrogen atom or a leaving group, Y₁ and Y₂ each independently represent an electron-attracting substituent, and R₁ represents an alkyl group, an aryl group, or a heterocyclic group.) ;

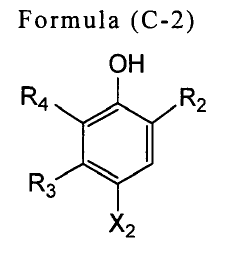

(wherein X₂ represents a hydrogen atom or a leaving group, R₂ represents an acylamino group, a ureido group, or a urethane group, R₃ represents a hydrogen atom, an alkyl group, or an acylamino group, R₄ represents a hydrogen atom or a substituent, and R₃ and R₄ may link together to form a ring.);

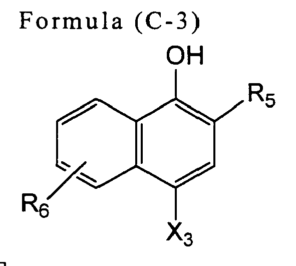

(wherein X₃ represents a hydrogen atom or a leaving group, R₅ represents a carbamoyl group or a sulfamoyl group, and R₆ represents a hydrogen atom or a substituent.);

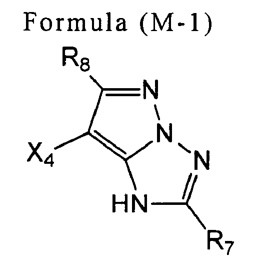

(wherein X₄ represents a hydrogen atom or a leaving group, R₇ represents an alkyl group, an aryl group, or a heterocyclic group, and R₈ represents a substituent.);

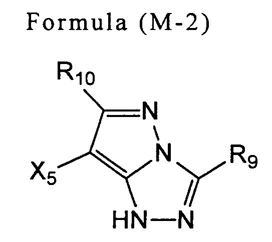

(wherein X₅ represents a hydrogen atom or a leaving group, R₉ represents an alkyl group, an aryl group, or a heterocyclic group, and R₁₀ represents a substituent.);

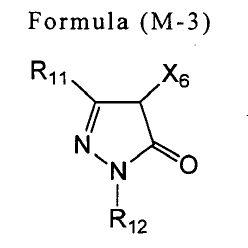

(wherein X₆ represents a hydrogen atom or a leaving group, R₁₁ represents an alkyl group, an aryl group, an acylamino group, or an anilino group, and R₁₂ represents an alkyl group, an aryl group, or a heterocyclic group.);

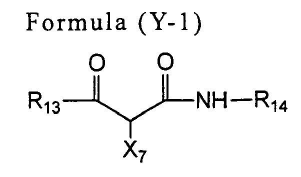

(wherein X₇ represents a hydrogen atom or a leaving group, R₁₃ represents an alkyl group, an aryl group, or an indolenyl group, and R₁₄ represents an aryl group or a heterocyclic group.);

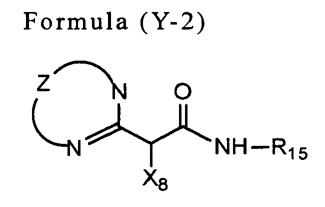

(wherein X₈ represents a hydrogen atom or a leaving group, Z represents a divalent group necessary for forming a 5- to 7-membered ring, and R₁₅ represents an aryl group or a heterocyclic group.);

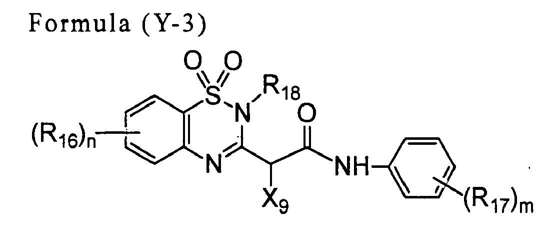

(wherein X₉ represents a hydrogen atom or a leaving group, R₁₆, R₁₇, and R₁₈ each independently represent a substituent, n represents an integer of from 0 to 4, and m represents an integer of from 0 to 5, when n represents 2 or more, a plurality of R₁₆ may be the same or different from one another, and when m represents 2 or more, a plurality of R₁₇ may be the same or different from one another.).

In formula (C-1), X₁ represents a hydrogen atom or a leaving group, and Y₁ and Y₂ each independently represent an electron-attracting substituent. R₁ represents an alkyl group, an aryl group, or a heterocyclic group, each of which may have a substituent.
X₁ is a hydrogen atom or a leaving group, and preferably a leaving group.

The leaving group in the present invention means the group which is possible to leave from the skeleton at the time of formation of dye by coupling with an oxidation product of a color developing agent. As the leaving group, a halogen atom, an alkoxy group, an aryloxy group, an alkylthio group, an arylthio group, an acyloxy group, a carbamoyloxy group, an imido group, a methylol group, a heterocyclic group, and the like are described. X₁ is more preferably a carbamoyloxy group or a benzoyloxy group. Y₁ and Y₂ represent an electron-attracting group. Specifically, a cyano group, a nitro group, an acyl group, an oxycarbonyl group, a carbamoyl group, a sulfonyl group, a sulfoxide group, an oxysulfonyl group, a sulfamoyl group, a heterocyclic group, a trifluoromethyl group, and a halogen atom are described. Among these, a cyano group, an oxycarbonyl group, and a sulfonyl group are preferable, and a cyano group and an oxycarbonyl group are more preferable. Even more preferably, one of Y₁ or Y₂ is a cyano group, and particularly preferably, Y₁ is a cyano group. Y₂ is preferably an oxycarbonyl group and particularly preferably, Y₂ preferably is an oxycarbonyl group substituted by a bulky group (for example, 2,6-di-t-butyl-4-methylpiperazinylocycarbonyl group). R₁ is preferably an alkyl group or an aryl group, each of which may have a substituent. As the alkyl group, a secondary or tertiary alkyl group is preferable, and a tertiary alkyl group is more preferable. The alkyl group preferably has from 3 to 12 carbon atoms, and more preferably from 4 to 8 carbon atoms. As the aryl group, preferable is a phenyl group, which may have a substituent, and the aryl group preferably has from 6 to 16 carbon atoms, and more preferably from 6 to 12 carbon atoms. Concerning the dye precursor of formula (C-1), the molecular weight is preferably 700 or less, more preferably 650 or less, and even more preferably 600 or less.

In formula (C-2), X₂ represents a hydrogen atom or a leaving group, R₂ represents an acylamino group, a ureido group, or a urethane group, R₃ represents a hydrogen atom, an alkyl group, or an acylamino group, and R₄ represents a hydrogen atom or a substituent. R₃ and R₄ may link together to form a ring.

Although X₂ is a hydrogen atom or a leaving group similar to X₁, X₂ is preferably a halogen atom, an aryloxy group, an alkoxy group, an arylthio group, or an alkylthio group, and more preferably a halogen atom or an aryloxy group. R₂ is preferably an acylamino group or a ureido group. R₂ preferably has from 2 to 12 carbon atoms in total, and more preferably from 2 to 8 carbon atoms in total. R₃ is preferably an alkyl group having 1 to 4 carbon atoms or an acylamino group having 2 to 12 carbon atoms, and more preferably an alkyl group having 2 to 4 carbon atoms or an acylamino group having 2 to 8 carbon atoms. R₄ is preferably a halogen atom, an alkoxy group, an acylamino group, or an alkyl group, more preferably a halogen atom or an acylamino group, and particularly preferably a chlorine atom. Concerning the dye precursor of formula (C-2), the molecular weight is preferably 500 or less, more preferably 450 or less, and even more preferably 400 or less.

In formula (C-3), X₃ is a hydrogen atom or a leaving group similar to X₁, however X₃ is preferably a halogen atom, an aryloxy group, an alkoxy group, an arylthio group, or an alkylthio group, and more preferably an alkoxy group or an alkylthio group. R₅ is preferably an acyl group, an oxycarbonyl group, a carbamoyl group, or a sulfamoyl group, and more preferably a carbamoyl group or a sulfamoyl group. R₅ is preferably a group having 1 to 12 carbon atoms, and more preferably a group having 2 to 10 carbon atoms. R₆ is a hydrogen atom or a substituent, and the substituent is preferably an amido group, a sulfonamide group, a urethane group or a ureido group, and more preferably an amido group or a urethane group. As the substitution position, the 5th or 8th position of a naphthol ring is preferable and the 5th position is more preferable. R₆ is preferably a group having 2 to 10 carbon atoms, and more preferably a group having 2 to 6 carbon atoms. Concerning the dye precursor of formula (C-2), the molecular weight is preferably 550 or less, more preferably 500 or less, and even more preferably 450 or less.

In formula (M-1), X₄ is a hydrogen atom or a leaving group similar to X₁, however X₄ is preferably a halogen atom, an aryloxy group, an alkoxy group, an arylthio group, an alkylthio group, or a heterocyclic group, and more preferably a halogen atom, an aryloxy group, an arylthio group or a heterocyclic group. As the heterocyclic group, an azole group such as a pyrazole group, an imidazole group, a triazole group, a tetrazole group, a benzimidazole group, and a benzotriazole group are preferable, and a pyrazole group is more preferable. R₇ is an alkyl group, an aryl group, or a heterocyclic group, each of which may have a substituent. Preferable are a secondary or tertiary alkyl group and an aryl group. As the alkyl group, an alkyl group having 2 to 14 carbon atoms is preferred, and more preferred is an alkyl group having 3 to 10 carbon atoms. As the aryl group, an aryl group having 6 to 18 carbon atoms is preferred, and more preferred is an aryl group having 6 to 14 carbon atoms. R₈ is preferably an alkyl group, an aryl group, an alkoxy group, an aryloxy group, an alkylthio group, an arylthio group or a heterocyclic group, each of which may have a substituent. The alkyl group is preferably a secondary or tertiary alkyl group, and more preferably a tertiary alkyl group. The alkyl group preferably has from 3 to 12 carbon atoms, and more preferably from 4 to 8 carbon atoms. The aryl group is preferably a phenyl group, which may have a substituent, and preferably has from 6 to 16 carbon atoms, and more preferably from 6 to 12 carbon atoms. As the alkoxy group, an alkoxy group having 1 to 8 carbon atoms is preferable, and an alkoxy group having 1 to 4 carbon atoms is more preferable. As the aryloxy group, an aryloxy group having 6 to 14 carbon atoms is preferable, and an aryloxy group having 6 to 10 carbon atoms is more preferable. The alkylthio group and the arylthio group are preferably the groups having carbon atoms in a similar number to the alkoxy group and the aryloxy group, respectively. Concerning the dye precursor of formula (M-1), the molecular weight is preferably 600 or less, more preferably 550 or less, and even more preferably 500 or less.

The groups represented by X₅, R₉, and R₁₀ of the dye precursor of formula (M-2) are similar groups as those represented by X₄, R₇, and R₈ of the dye precursor of formula (M-1), respectively, and preferable range of each group of them is similar to that of the dye precursor of formula (M-1).

In formula (M-3), although X₆ is a hydrogen atom or a leaving group similar to X₁, X₆ is preferably an alkylthio group, an arylthio group, or a heterocyclic group, and more preferably an arylthio group or a heterocyclic group. As the arylthio group, a phenyl group is preferable, and more preferable is an arylthio group in which an alkoxy group or an amido group is substituted at the 2nd position.
The arylthio group preferably has from 6 to 16 carbon atoms, and more preferably from 7 to 12 carbon atoms. As the heterocyclic group, an azole group such as a pyrazole group, an imidazole group, a triazole group, a tetrazole group, a benzimidazole group, a benzotriazole group, or the like is preferable, and more preferable is a pyrazole group. As R₁₁, an alkyl group, an aryl group, an acylamino group, and an anilino group are preferable, and an acylamino group and an anilino group are more preferable. An anilino group is most preferable. As the alkyl group, an alkyl group having 1 to 8 carbon atoms is preferable; and as the aryl group, an aryl group having 6 to 14 carbon atoms is preferable. As the acylamino group, an acylamino group having 2 to 14 carbon atoms is preferable, and an acylamino group having 2 to 10 carbon atoms is more preferable. As the anilino group, an anilino group having 6 to 16 carbon atoms is preferable, and an anilino group having 6 to 12 carbon atoms is more preferable. As a substituent of the anilino group, a halogen atom and an acylamino group are preferable. Concerning the dye precursor of formula (M-3), the molecular weight is preferably 700 or less, more preferably 650 or less, and even more preferably 600 or less.

In formula (Y-1), although X₇ is a hydrogen atom or a leaving group similar to X₁, X₇ is preferably an aryloxy group, an imido group, or a heterocyclic group. As the aryloxy group, an aryloxy group which is substituted by an electron-attracting group is preferable. As the imido group, a cyclic imido group is preferable, and a hydantoin group, a 1,3-oxazolidine-2,5-dione group, and a succinimide group are particularly preferable. The imido group preferably has from 3 to 15 carbon atoms in total, more preferably from 4 to 11 carbon atoms in total, and even more preferably from 5 to 9 carbon atoms in total. As the heterocyclic group, a pyrazole group, an imidazole group, a triazole group, a tetrazole, a benzimidazole group, and a benzotriazole group are preferable, and an imidazole group is more preferable. The azole group preferably has from 3 to 12 carbon atoms in total, more preferably from 3 to 10 carbon atoms in total, and even more preferably from 3 to 8 carbon atoms in total. R₁₃ is preferably a secondary or tertiary alkyl group, an aryl group, or a heterocyclic group. The alkyl group may be a cycloalkyl group or a bicycloalkyl group, and a tertiary alkyl group is preferable. A 1-alkylcyclopropyl group, a bicycloalkyl group, and an adamantyl group are particularly preferable. R₁₄ is preferably an aryl group or a heterocyclic group, and more preferably an aryl group. Among them, a phenyl group substituted by a halogen atom, an alkoxy group, an aryloxy group, an alkylthio group, or an arylthio group at the 2nd position is particularly preferable. R₁₄ preferably has from 6 to 18 carbon atoms in total, more preferably from 7 to 16 carbon atoms in total, and even more preferably from 8 to 14 carbon atoms. Concerning the dye precursor of formula (Y-1), the molecular weight is preferably 700 or less, more preferably 650 or less, and even more preferably 600 or less.

The groups represented by X₈ and R₁₅ in the dye precursor of formula (Y-2) are similar to the groups represented by X₇ and R₁₄ in the dye precursor of formula (Y-1) respectively, and preferable range of each group of them is similar to that of the dye precursor of formula (Y-1). Z represents a divalent group necessary to form a 5-to 7-membered ring, and this ring may have a substituent or may be condensed by another ring.

Among the dye precursors of formula (Y-2), the dye precursor represented by formula (Y-3) is preferable.
In the dye precursor of formula (Y-3), X₉ has the same meaning as X₇ in formula (Y-1), and its preferable range is also the same. R₁₆ is preferably a halogen atom, an alkyl group, an alkoxy group, an acyl group, an acyloxy group, an acylamino group, an alkoxycarbonyl group, a sulfonamide group, a cyano group, a sulfonyl group, a sulfamoyl group, a carbamoyl group, or an alkylthio group, and more preferably a substituent having 1 to 4 carbon atoms. n is preferably an integer of from 0 to 3, more preferably an integer of from 0 to 2, even more preferably 0 or 1, and most preferably zero. R₁₇ is preferably a group similar to R₁₆, and more preferably a halogen atom, an alkyl group, an alkoxy group, an acylamino group, a sulfonamide group, an alkoxycarbonyl group, a sulfamoyl group, or a sulfonyl group. R₁₇ is particularly preferably a halogen atom, an alkoxy group, or an alkylthio group which substitutes at the ortho position with respect to the -NH- group. An alkylthio group is most preferable.
The molecular weight of the dye precursor of formula (Y-3) is preferably 750 or less, more preferably 700 or less, and even more preferably 650 or less.

Specific examples of the dye precursor of the present invention are described below, but the present invention is not limited to these compounds.

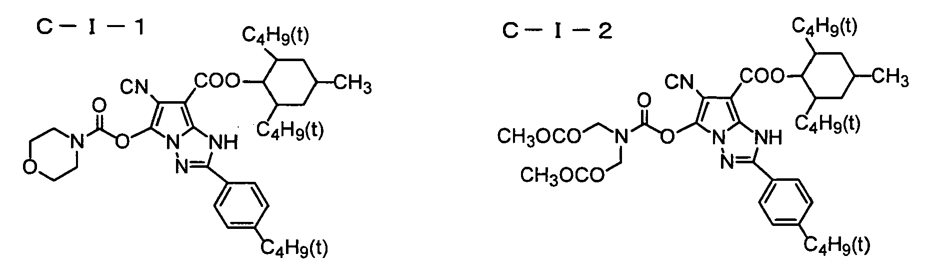

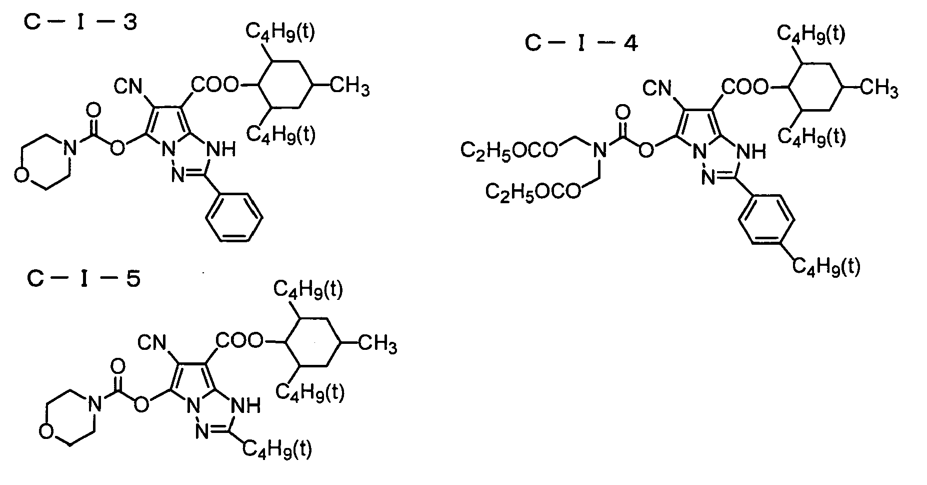

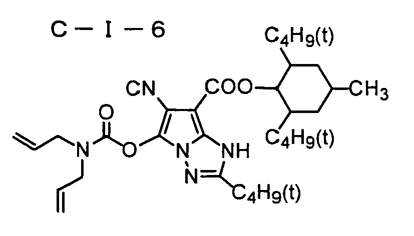

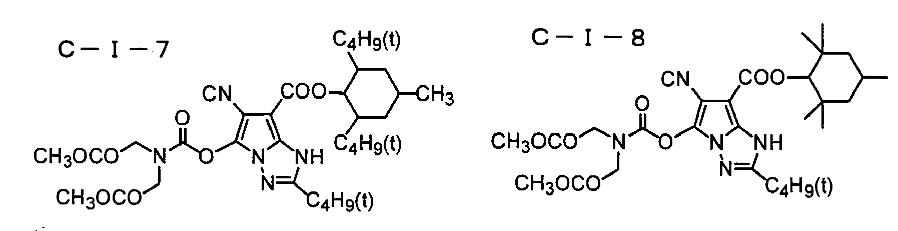

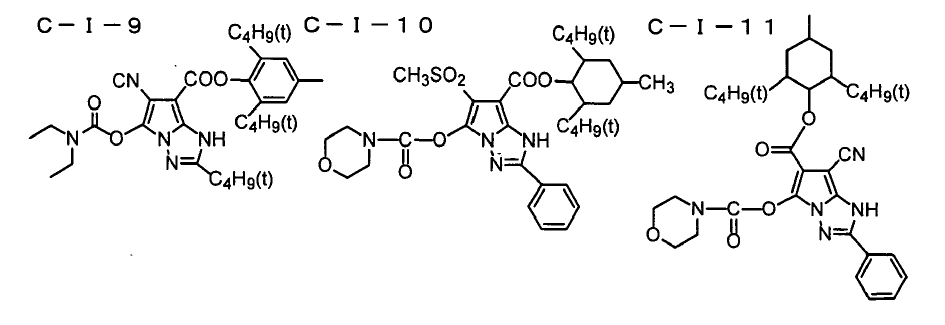

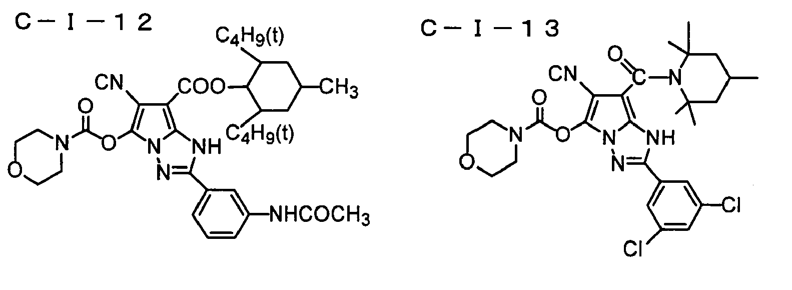

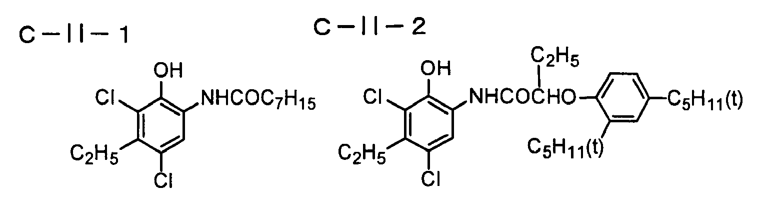

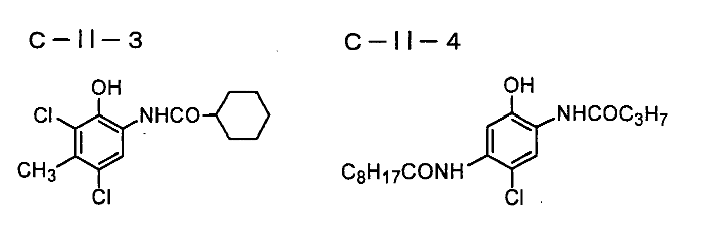

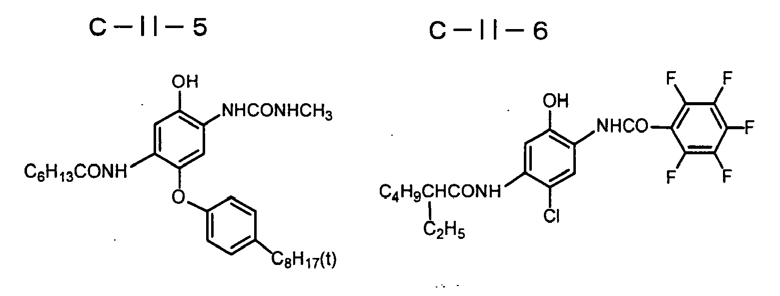

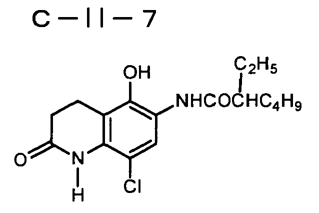

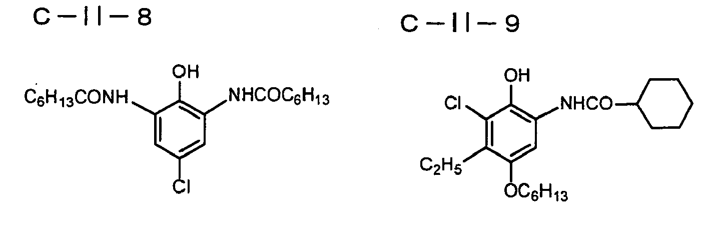

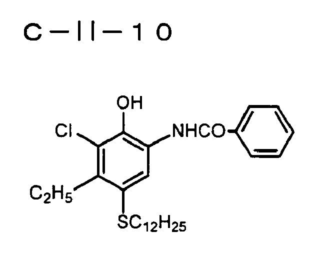

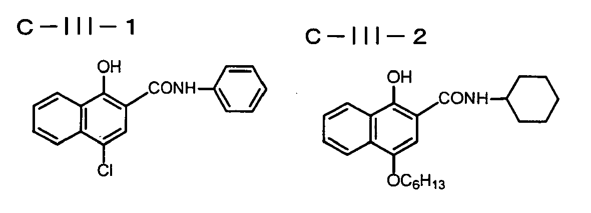

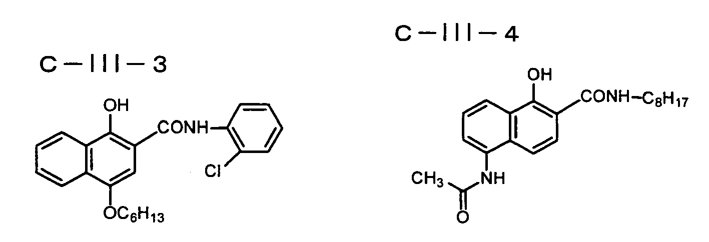

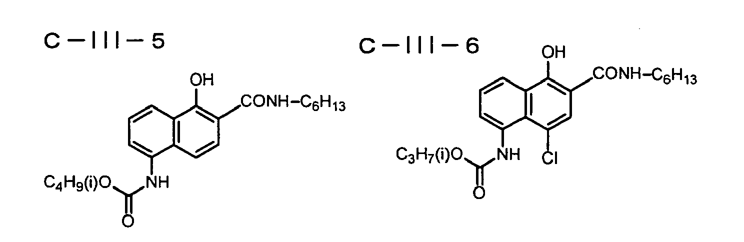

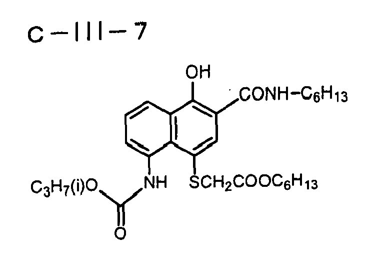

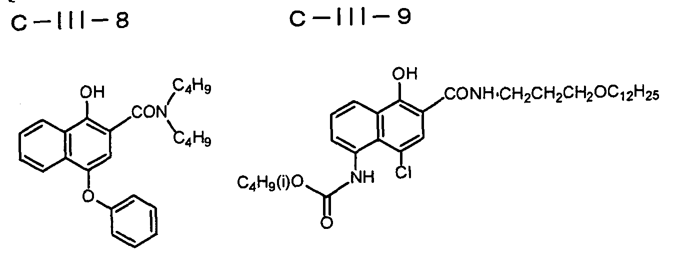

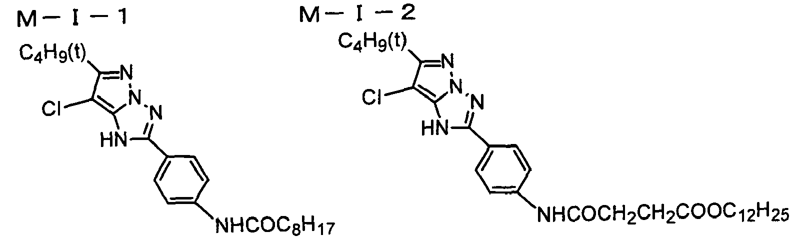

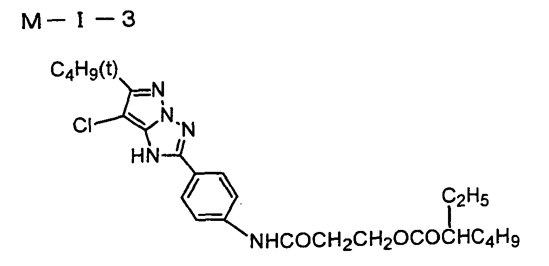

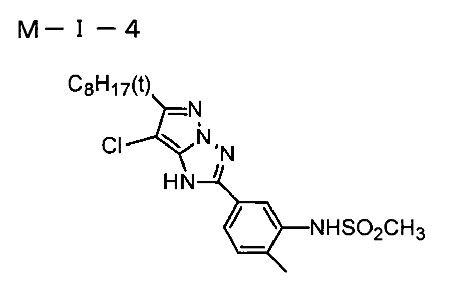

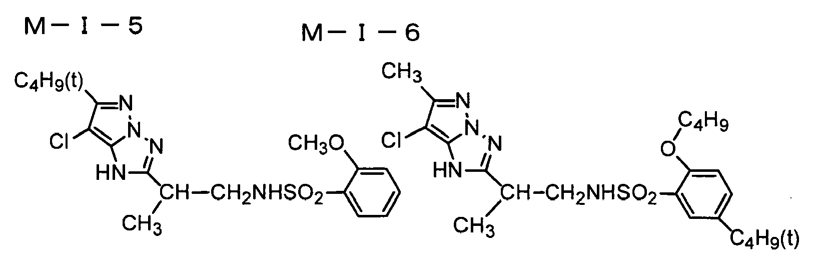

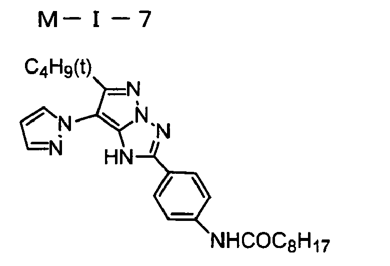

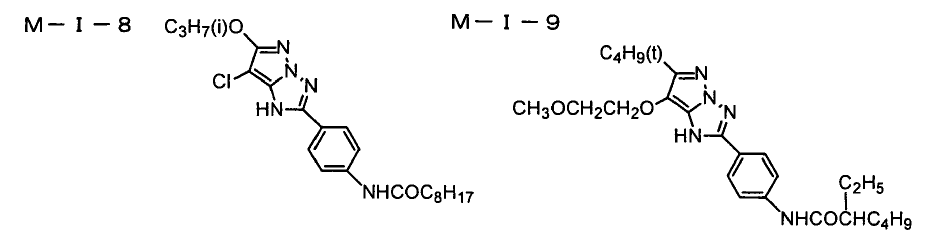

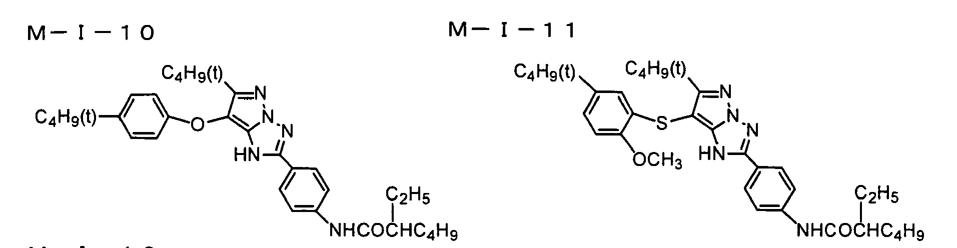

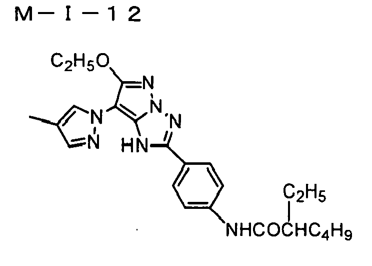

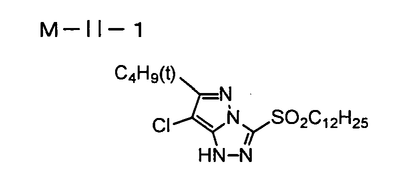

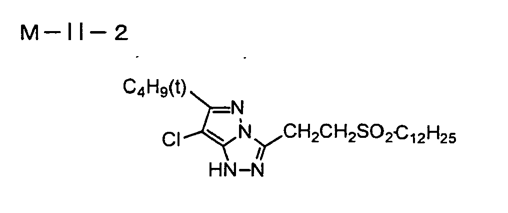

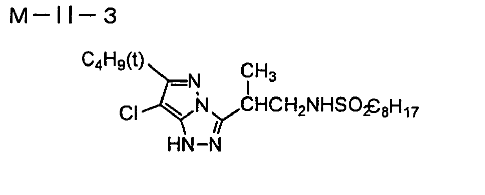

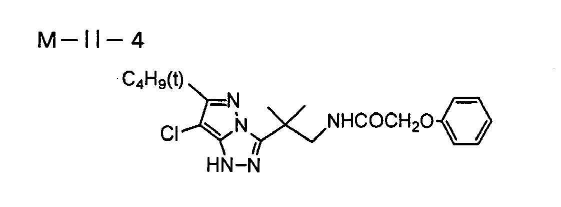

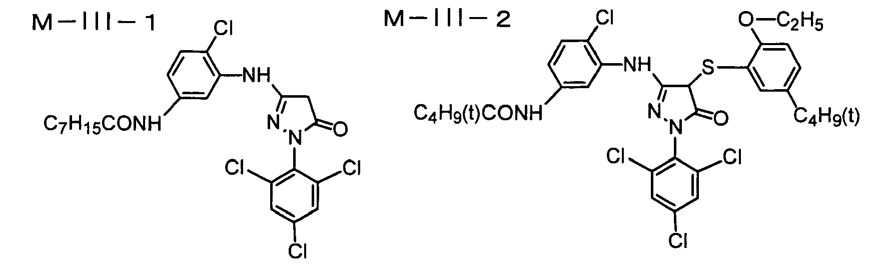

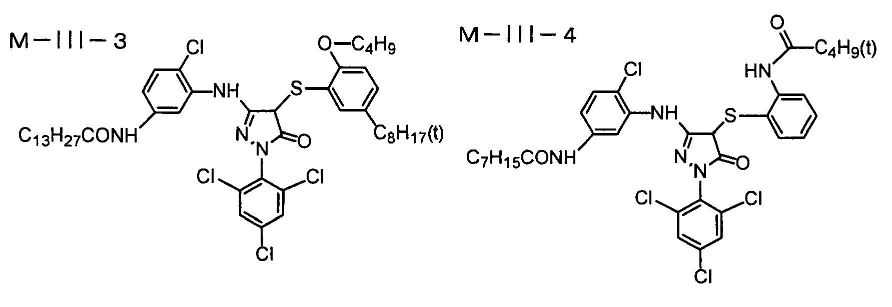

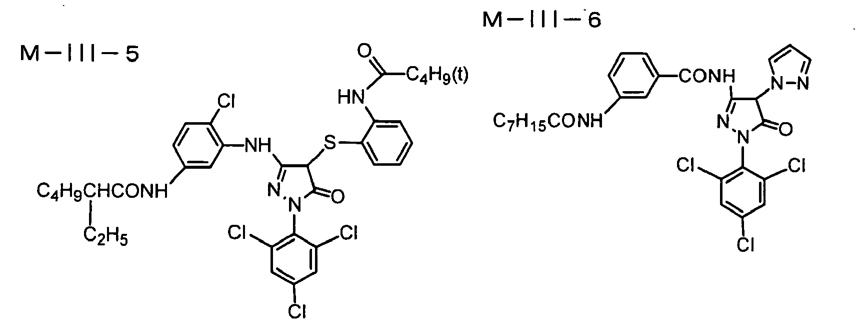

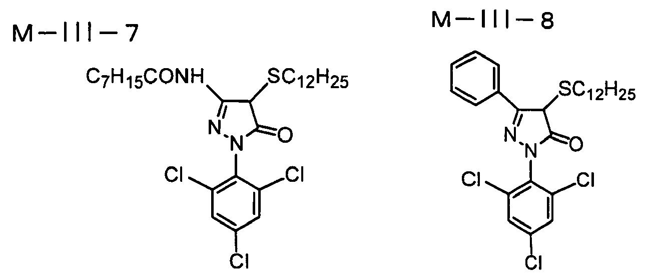

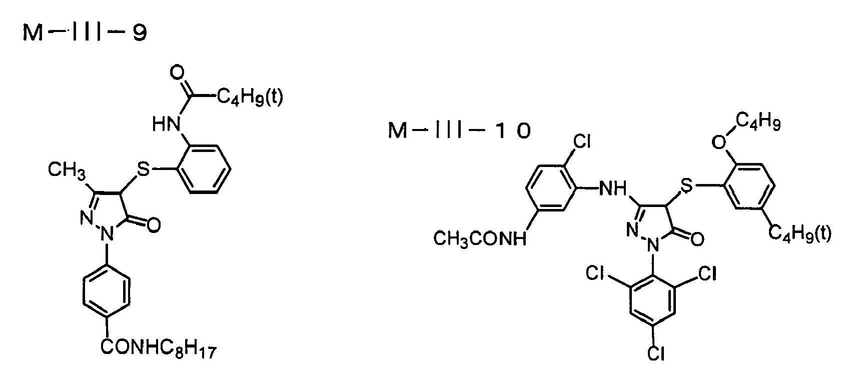

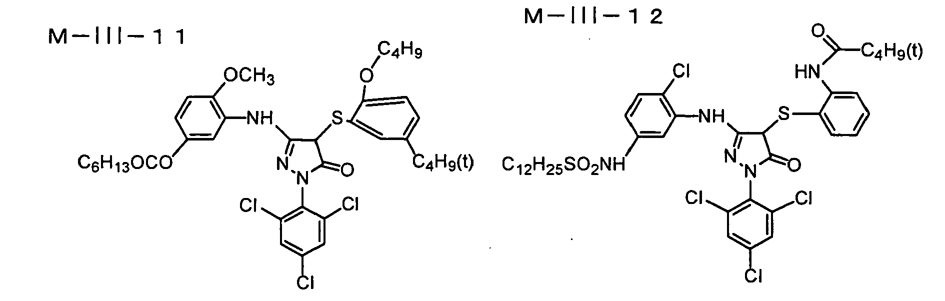

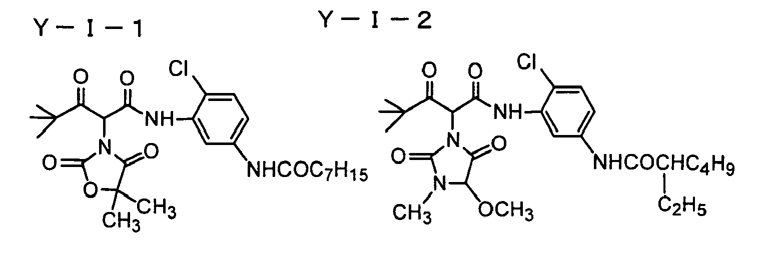

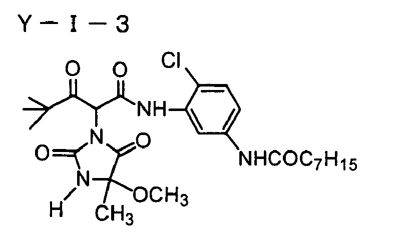

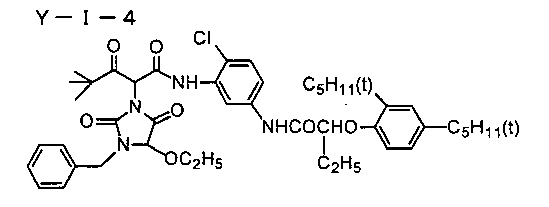

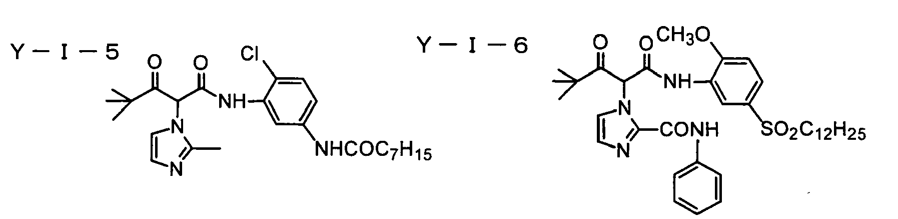

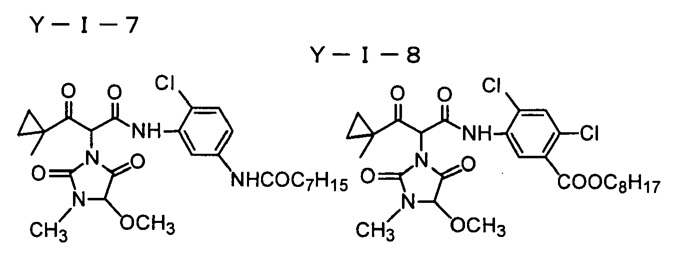

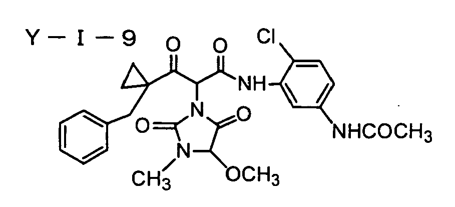

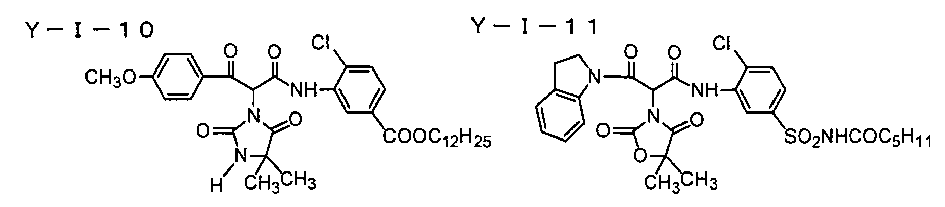

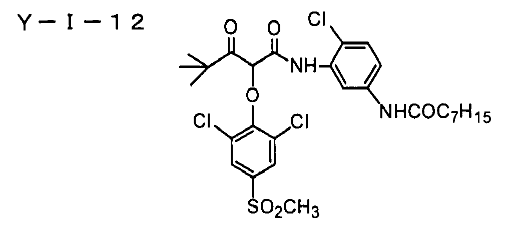

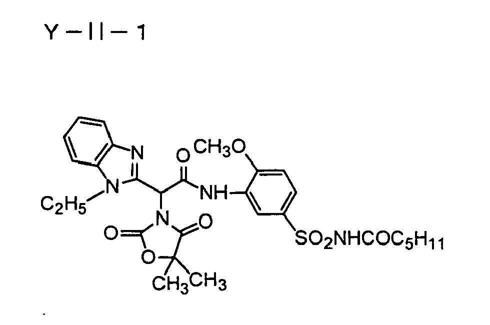

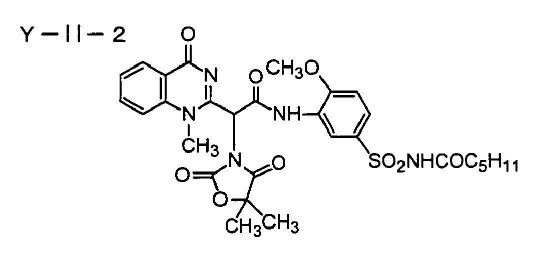

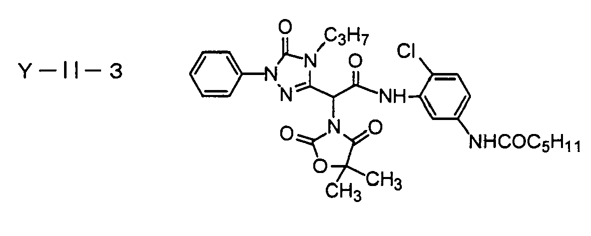

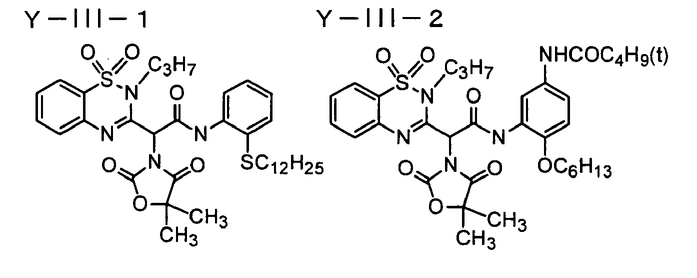

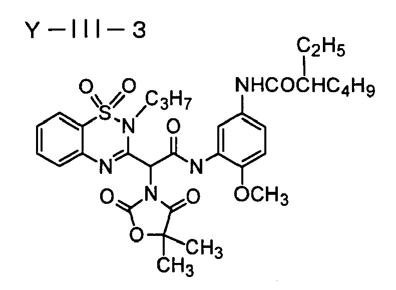

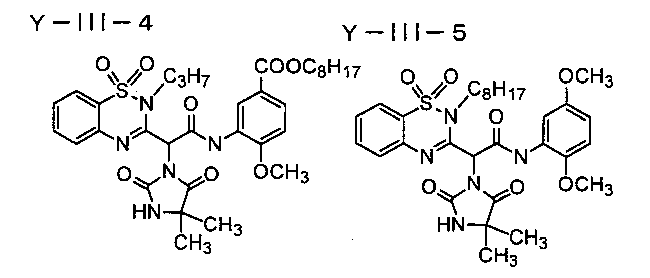

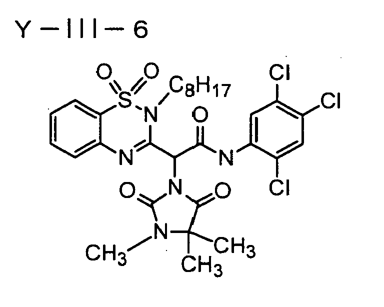

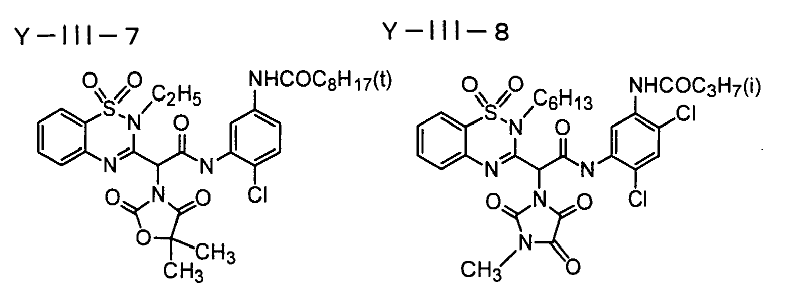

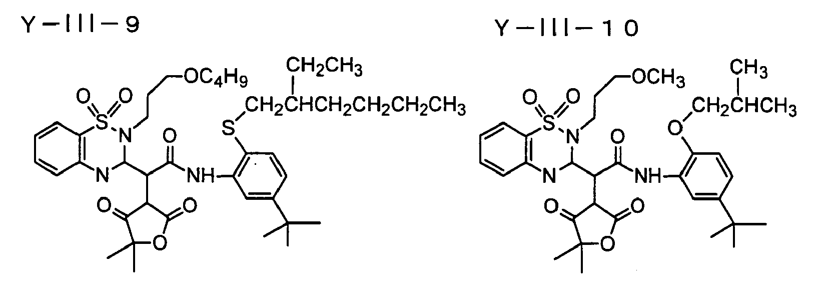

Other than the above compounds, compound Nos. CP101 to CP117, CP201 to CP220, and CP301 to CP331 described in JP-A No. 2004-4439 and the like can also be used in the invention.

The dye precursor of the present invention can be used as an oil emulsion or a solid particle dispersion, for example, it can be added to a coating film using gelatin or polymer latex as a binder. In this case, the coating amount is preferably from 1 × 10⁻⁶ mol/m² to 1 × 10⁻² mol/m², and more preferably from 1 × 10⁻⁵ mol/m² to 1 × 10⁻³ mol/m².

The layer containing the protease substrate according to the present invention is thinner than the layer containing the dye precursor, and preferably has a thickness of 3 µm or less and more preferably 1 µm or less.

The layer containing the dye precursor according to the present invention is thicker than the layer containing the protease substrate, and preferably has a thickness of from 1 µm to 30 µm and more preferably from 3 µm to 20 µm.

4) Color forming method In the present invention, the color developing agent may be included in the layer containing the dye precursor or the layer containing the protease substrate, or another layer containing the color developing agent may be disposed on the same side of the support as the layer containing the dye precursor or the layer containing the protease substrate. Otherwise, a method of coating a processing solution including the color developing agent, after the formation of a trace of digestion, can be adopted.

### (Hardener)

It is preferred that the layer containing the protease substrate according to the present invention includes gelatin as a binder, and further, a gelatin hardener.
As the hardener, a material known as a hardener for gelatin in the photographic use can be applied. The hardener is added in an amount of from 0.2% by weight to 3.0% by weight with respect to the coating amount of gelatin in the layer containing a protease substrate, and more preferably from 0.5% by weight to 1.0% by weight.

### (Cell staining)

The invention can use cell staining in combination. When performing cell staining, so as to be distinguishable from a color-forming dye, the use of a dye having different hue is preferable. Further, a biological sample on the thin film may be subjected to tissue staining with a dye having different color tone to compare traces of digestion and the stained tissue. For example, when the cell nuclei of cells contained in a biological sample is stained with hematoxylin or methyl green, morphological change of the nuclei and traces of digestion can simultaneously be observed as signals having different color tones under an optical microscope. Furthermore, by combining the tissue staining method and the method using a protease inhibitor, the existence and the identification of the type of protease localized in respective cells can also be performed at the same time.

By measuring the protease activity included in a sample in accordance with the methods of the invention, the state of a living body from which the sample has been separated, for example, metabasis of cancer or the degree of progress in rheumatism can be examined. Therefore, the methods of the invention can be utilized for various purposes such as diagnosis of diseases, clinical assay and animal testing. For judging the intensity of digestion in traces of digestion, such methods can be utilized as making judgment visually under an optical microscope, measuring an optical density of traces of digestion using a spectrometer, and importing images obtained using an optical microscope into a computer and performing various kinds of digitization for traces of digestion by a method of image analysis.

### EXAMPLES

In the following, the present invention will be explained by examples thereof, but the present invention is by no means limited by such examples.

### EXAMPLE 1

### 1. Preparation of Layer Containing Dye Precursor (Color-Forming Layer)

4.5 g of a coupler (CP-1), 1.3 g of a color image stabilizer (ST-1), 0.22 g of a color image stabilizer (ST-2), and 1.4 g of a color image stabilizer (ST-3) were dissolved in a mixture of 6.7 g of a solvent (SOL-1), 6.7 g of a solvent (SOL-2) and 19 g of a solvent (ethyl acetate). The resulting solution was mixed with 84 mL of a 25% by weight aqueous solution of alkali-processed bovine bone gelatin containing 1.0 g of sodium dodecylbenzenesulfonate, which was emulsified and dispersed using a high-speed stirring emulsifier (dissolver), to which water was further added to prepare 230 g of an emulsified dispersion A.

To the emulsified dispersion A, an aqueous solution of alkali-processed bovine bone gelatin was added so as to give a coating solution having a composition below, which was then coated on a triacetyl cellulose (TAC) support and dried to prepare a color-forming layer.

| | (g/m²) |
|---|---|
| CP-1 | 0.27 |
| ST-1 | 0.079 |
| ST-2 | 0.0133 |
| ST-3 | 0.085 |
| SOL-1 | 0.41 |
| SOL-2 | 0.41 |
| Alkali-processed bovine bone gelatin | 2.2 |

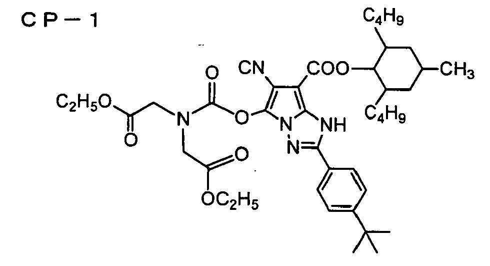

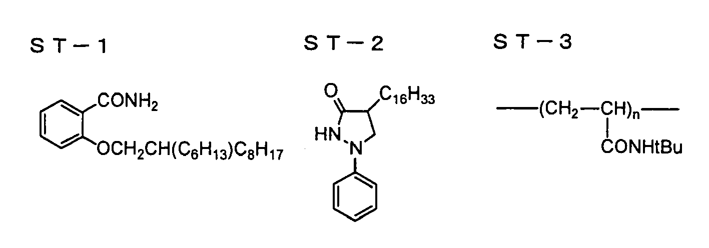

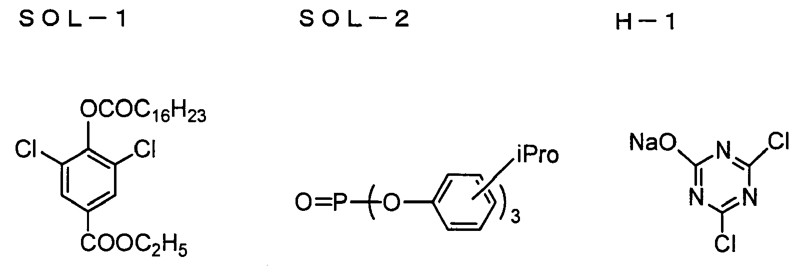

### 2. Preparation of Layer Containing Protease Substrate (Substrate Layer)

A coating liquid was prepared so as to give the following composition including acid-processed swine cutis gelatin to be a protease substrate, a hardener (M-1), and silver particles EMSC-60 to be a development center-forming substance manufactured by BBI.
The liquid was coated on a sheet coated with the above-described color-forming layer and was dried to prepare a protease detection material provided with a substrate layer. In this process, protease detection materials were prepared so that the thickness of the substrate layer gave 0.5 µm, 1.0 µm, 2.0 µm, 3.0 µm, or 4.0 µm.

**<Substrate layer 0.5 µm>**

| | (g/m²) |
|---|---|
| Acid-processed swine cutis gelatin | 0.672 |
| H-1 | 0.0162 |
| Silver fine particles | 0.0012 |

**<Substrate layer 1.0 µm>**

| | (g/m²) |
|---|---|
| Acid-processed swine cutis gelatin | 1.343 |
| H-1 | 0.0324 |
| Silver fine particles | 0.0012 |

**<Substrate layer 2.0 µm>**

| | (g/m²) |
|---|---|
| Acid-processed swine cutis gelatin | 2.686 |
| H-1 | 0.0648 |
| Silver fine particles | 0.0012 |

**<Substrate layer 3.0 µm>**

| | (g/m²) |
|---|---|
| Acid-processed swine cutis gelatin | 4.029 |
| H-1 | 0.0972 |
| Silver fine particles | 0.0012 |

**<Substrate layer 4.0 µm>**

| | (g/m²) |
|---|---|
| Acid-processed swine cutis gelatin | 5.372 |
| H-1 | 0.130 |
| Silver fine particles | 0.0012 |

To the above-described protease detection material, 10 µL of a solution prepared by dissolving papain, which is a protease, in a 0.5 M phosphate buffer solution was dropped, which was incubated at 37°C for 30 minutes or 5 hours under an environment of 100% relative humidity. The concentration of the papain was 1 units/mL, 0.5 units/mL, 0.25 units/mL, 0.13 units/mL, or 0.06 units/mL.
After the lapse of a predetermined time, the dropped papain solution was washed with pure water.

### Preparation of Color-Forming Solution

To 800 mL of an aqueous buffer solution containing 0.4 mL of an 8 N potassium hydroxide solution and 26.4 g of potassium hydrogen carbonate, 4 g of potassium sulfite and 3.2 g of a color developing agent (I-12) represented by formula (I) were added and dissolved, which was diluted with water to give the whole volume of 1000 mL, thereby preparing a color-forming solution.

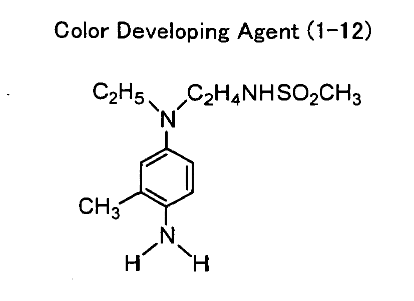

Into a liquid prepared by adding 1.6 mL of 30% by weight solution of hydrogen peroxide to 48 mL of the color-forming solution, the incubated protease detection material was immersed for 10 minutes to develop. Portions where the substrate layer had been decomposed by the protease were decolored to be white, and other portions formed color in cyan.

### (Comparative Example)

A coating solution was prepared so as to give the following composition including acid-processed swine cutis gelatin, which is a protease substrate, and the hardener (H-1), which was coated on a TAC support and dried to prepare a protease detection material. The protease detection materials were prepared so that the thickness of the substrate layer gave 4.0 µm, 5.0 µm, 6.0 µm, 7.0 µm, or 8.0 µm.

**<Coated film 4.0 µm>**

| | (g/m²) |
|---|---|
| Acid-processed swine cutis gelatin | 5.372 |
| H-1 | 0.130 |

**<Coated film 5.0 µm>**

| | (g/m²) |
|---|---|
| Acid-processed swine cutis gelatin | 6.715 |
| H-1 | 0.162 |

**<Coated film 6.0 µm>**

| | (g/m²) |
|---|---|
| Acid-processed swine cutis gelatin | 8.058 |
| H-1 | 0.194 |

**<Coated film 7.0 µm>**

| | (g/m²) |
|---|---|
| Acid-processed swine cutis gelatin | 9.401 |
| H-1 | 0.2268 |

**<Coated film 8.0 µm>**

| | (g/m²) |
|---|---|
| Acid-processed swine cutis gelatin | 10.744 |
| H-1 | 0.2592 |

To the above-described protease detection material, 10 µL of a solution prepared by dissolving papain, which is a protease, in a 0.5 M phosphate buffer solution was dropped, which was incubated at 37°C for 30 minutes or 5 hours under an environment of 100% relative humidity. The concentration of the papain was 1 units/mL, 0.5 units/mL, 0.25 units/mL, 0.13 units/mL, or 0.06 units/mL. After the lapse of a predetermined time, the dropped papain solution was washed with pure water.

While using a solution prepared by dissolving Ponceau 3R so as to give a concentration of 0.8% in a 6% aqueous solution of trichloroacetic acid as a staining solution, the incubated protease detection material was immersed at room temperature for 60 seconds for staining. The material was immersed in water for ten minutes to be washed.

### Evaluation

### <Evaluation of Detection Sensitivity>

Portions of developed and stained sample, onto which papain solutions of respective concentrations had been dropped, were visually observed and judged based on the following evaluation standard;
○: color is clearly removed to be distinguishable;
×: decoloration is unclear, or color is not removed entirely.

### <Evaluation of Image Quality>

In the case where observation is performed using an optical microscope, generally, distinct contrast cannot be obtained when the transmission optical density (visual) is lower than 1.0. The color density at color-formed portions was judged based on the following evaluation standard;
good: the transmission optical density of the color-formed portion is 1.0 or more;
poor: the transmission optical density of the color-formed portion is lower than 1.0.

From the above evaluation results, it is understood that the invention makes it possible to detect the decomposition of a substrate by protease in very short time with high sensitivity.

**TABLE 1**

| <Incubation for 30 minutes> | | Evaluation of Detection Sensitivity | | | | | |
|---|---|---|---|---|---|---|---|
| | | Concentration of Added Papain (units/mL) | | | | | |
| | Thickness of Substrate Layer (µm) | 1 | 0.5 | 0.25 | 0.13 | 0.06 | Evaluation of Image Quality |
| Inventive Example | 0.5 | ○ | ○ | ○ | ○ | × | good |
| | 1.0 | ○ | ○ | ○ | × | × | good |
| | 2.0 | ○ | ○ | ○ | × | × | good |
| | 3.0 | ○ | ○ | × | × | × | good |
| | 4.0 | ○ | × | × | × | × | good |
| Comparative Example | 4.0 | ○ | × | × | × | × | poor |
| | 5.0 | × | × | × | × | × | poor |
| | 6.0 | × | × | × | × | × | good |
| | 7.0 | × | × | × | × | × | good |
| | 8.0 | × | × | × | × | × | good |
| | | | | | | | |

| <Incubation for 5 hours> | | Evaluation of Detection Sensitivity | | | | | |
|---|---|---|---|---|---|---|---|
| | | Concentration of Added Papain (units/mL) | | | | | |
| | Thickness of Substrate Layer (µm) | 1 | 0.5 | 0.25 | 0.13 | 0.06 | Evaluation of Image Quality |
| Inventive Example | 0.5 | ○ | ○ | ○ | ○ | ○ | good |
| | 1.0 | ○ | ○ | ○ | ○ | ○ | good |
| | 2.0 | ○ | ○ | ○ | ○ | × | good |
| | 3.0 | ○ | ○ | ○ | ○ | × | good |
| | 4.0 | ○ | ○ | ○ | × | × | good |
| Comparative Example | 4.0 | ○ | ○ | ○ | × | × | poor |
| | 5.0 | ○ | ○ | ○ | × | × | poor |
| | 6.0 | ○ | ○ | × | × | × | good |
| | 7.0 | ○ | × | × | × | × | good |
| | 8.0 | ○ | × | × | × | × | good |

## Claims

1. A protease detection material comprising, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance.

2. The protease detection material according to claim 1, wherein the development center-forming substance comprises silver colloid, gold colloid, or silver halide.

3. The protease detection material according to claim 1, wherein a thickness of the layer containing the protease substrate is 3 µm or less.

4. The protease detection material according to claim 1, wherein the layer containing the protease substrate further contains a hardener.

5. The protease detection material according to claim 1, further comprising a color developing agent.

6. The protease detection material according to claim 5, wherein the color developing agent is a compound represented by the following formula (I): 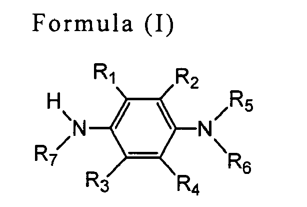 wherein R₁, R₂, R₃ and R₄ each independently represent a hydrogen atom or a substituent; R₅ and R₆ each independently represent an alkyl group, an aryl group, a heterocyclic group, an acyl group, or a sulfonyl group; R₁ and R₂, R₃ and R₄, R₅ and R₆, R₂ and R₅, or R₄ and R₆ may bond to each other in each combination to form a 5-, 6-, or 7-membered ring; R₇ represents a hydrogen atom, R₁₁-O-CO-, R₁₂-COCO-, R₁₃-NH-CO-, R₁₄-SO₂-, R₁₅-W-C(R₁₆)(R₁₇)(R₁₈)-, R₁₉-SO₂NHCO-, R₂₀-CONHCO-, R₂₁-SO₂NHSO₂-, R₂₂-CONHSO₂-, or (M)_{1/n}OSO₂-; R₁₁, R₁₂, R₁₃, R₁₄, R₁₉, R₂₀, R₂₁, and R₂₂ each independently represent an alkyl group, an aryl group, or a heterocyclic group; R₁₅ represents a hydrogen atom or a block group; W represents an oxygen atom, a sulfur atom, or -N(R₁₈)-; R₁₆, R₁₇, and R₁₈ each independently represent a hydrogen atom or an alkyl group; and M represents a cation having a valency of n.

7. The protease detection material according to claim 5, wherein the color developing agent is a compound represented by the following formula (II):
Formula (II) R¹¹-NH-NH-X-R¹²
wherein in formula (II), R¹¹ represents an aryl group or a heterocyclic group, each of which may have a substituent; R¹² represents an alkyl group, an alkenyl group, an alkynyl group, an aryl group, or a heterocyclic group, each of which may have a substituent; X represents -SO₂-, -CO-, -COCO-, -CO-O-, -CO-N(R¹³)-, -COCO-O-, - COCO-N(R¹³)-, or -SO₂-N(R¹³)-; and R¹³ represents a hydrogen atom or a group selected from the groups that may be represented by R¹².

8. The protease detection material according to claim 7, wherein the color developing agent is a compound represented by the following formula (IV):
Formula (IV) R¹¹-NH-NH-CO-R¹²
wherein in formula (IV), R¹¹ and R¹² each have the same meaning as that in formula (II).

9. The protease detection material according to claim 1, wherein the dye precursor comprises at least one compound represented by a formula selected from the group consisting of the following formulae (C-1), (C-2), (C-3), (M-1), (M-2), (M-3), (Y-1), (Y-2), and (Y-3): 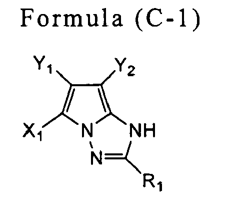 wherein X₁ represents a hydrogen atom or a leaving group; Y₁ and Y₂ each independently represent an electron-attracting substituent; and R₁ represents an alkyl group, an aryl group, or a heterocyclic group; 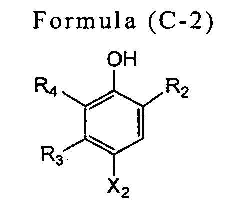 wherein X₂ represents a hydrogen atom or a leaving group; R₂ represents an acylamino group, a ureido group, or a urethane group; R₃ represents a hydrogen atom, an alkyl group, or an acylamino group; R₄ represents a hydrogen atom or a substituent; and R₃ and R₄ may link together to form a ring; 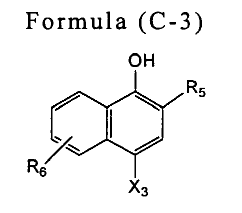 wherein X₃ represents a hydrogen atom or a leaving group; R₅ represents a carbamoyl group or a sulfamoyl group; and R₆ represents a hydrogen atom or a substituent; 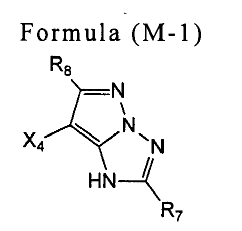 wherein X₄ represents a hydrogen atom or a leaving group; R₇ represents an alkyl group, an aryl group, or a heterocyclic group; and R₈ represents a substituent; 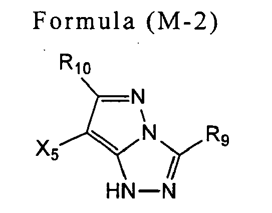 wherein X₅ represents a hydrogen atom or a leaving group; R₉ represents an alkyl group, an aryl group, or a heterocyclic group; and R₁₀ represents a substituent; 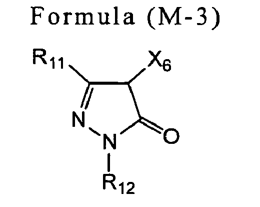 wherein X₆ represents a hydrogen atom or a leaving group; R₁₁ represents an alkyl group, an aryl group, an acylamino group, or an anilino group; and R₁₂ represents an alkyl group, an aryl group, or a heterocyclic group; 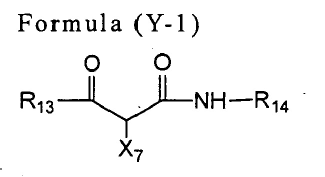 wherein X₇ represents a hydrogen atom or a leaving group; R₁₃ represents an alkyl group, an aryl group, or an indolenyl group; and R₁₄ represents an aryl group or a heterocyclic group; 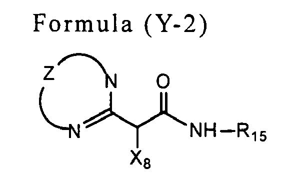 wherein X₈ represents a hydrogen atom or a leaving group; Z represents a divalent group necessary for forming a 5- to 7-membered ring; and R₁₅ represents an aryl group or a heterocyclic group; 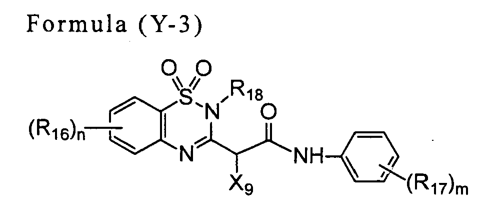 wherein X₉ represents a hydrogen atom or a leaving group; R₁₆, R₁₇, and R₁₈ each independently represent a substituent; n represents an integer of from 0 to 4; m represents an integer of from 0 to 5; when n represents 2 or more, a plurality of R₁₆ may be the same or different from one another; and when m represents 2 or more, a plurality of R₁₇ may be the same or different from one another.

10. A method for measuring protease using a protease detection material comprising, on a support, at least a layer containing a dye precursor and a layer which contains a protease substrate and is disposed on the same side of the support as the layer containing the dye precursor and farther from the support than the layer containing the dye precursor, wherein the layer containing the protease substrate further contains a development center-forming substance, the method comprising:
(1) bringing a biological test sample into contact with a surface of the protease detection material; and
(2) causing a developing agent to act on the surface of the protease detection material having been subjected to (1) to detect a trace of digestion by protease.

11. A set of protease detection materials comprising:
a first protease detection material having, on a first support, a first layer containing a dye precursor and a layer which contains a protease substrate and is disposed on the same side of the first support as the first layer containing the dye precursor and farther from the first support than the first layer containing the dye precursor, wherein the layer containing the protease substrate further contains a first development center-forming substance; and
a second protease detection material for reference having, on a second support, at least a second layer containing a dye precursor and a layer which contains a protease inhibitor for an object to be detected and is disposed on the same side of the second support as the second layer containing the dye precursor and farther from the second support than the second layer containing the dye precursor, wherein the layer containing the protease inhibitor for an object to be detected further contains a second development center-forming substance.

12. A method for measuring protease using the set of protease detection materials according to claim 11, the method comprising:
(1) bringing one slice among two or more substantially continuous slices of a biological test sample into contact with a surface of the first protease detection material;
(2) bringing another slice among the two or more continuous slices into contact with a surface of the second protease detection material for reference;
(3) causing a color developing agent to act on the surface of the first protease detection material having been subjected to (1) and on the surface of the second protease detection material for reference having been subjected to (2), to detect a trace of digestion by protease by means of color-formation pattern; and
(4) comparing the color-formation pattern of the first protease detection material with the color-formation pattern of the second protease detection material for reference.
